# EUROPEAN PATENT SPECIFICATION

(11) **EP 1 488 747 B1**
(45) Date of publication and mention of the grant of the patent: **01.04.2009**
(21) Application number: 04076655.2
(22) Date of filing: 09.04.1999
(51) Int. Cl.: A61B 17/17

(54) **Instrumentation for vertebral interbody fusion**
Instrumentarium zur Wirbelkörperfusion
Instrumentation pour arthrodese intersomatique du rachis

(30) Priority: 09.04.1998 US 81206 P
(43) Date of publication of application: 22.12.2004
(62) Divisional of application: 99919809.6
(73) Proprietor: Warsaw Orthopedic, Inc., Warsaw, Indiana 46581 (US)
(72) Inventor: Boyd, Lawrence M., Durham NC 27707 (US); Ray, Eddi F., Collierville TN 38017 (US); Estes, Bradley T., Durham NC 27705 (US); Liu, Mingyan, Bourg-la-Reine, France 92340 (FR)
(74) Representative: Dauncey, Mark Peter

(56) References cited:
- WO-A-93/19678
- WO-A-96/25103
- WO-A-96/27321
- WO-A-96/27345
- US-A- 5 669 915

## Description

The present invention relates generally to surgical procedures for spinal stabilization and more specifically to instrumentation adapted for inserting a spinal implant within the intervertebral disc space between adjacent vertebra. More particularly, while aspects of the invention may have other applications, the present invention is especially suited for disc space preparation and implant insertion into a disc space from a generally anterior approach to the spine.

Various surgical methods have been devised for the implantation of fusion devices into the disc space. Both anterior and posterior surgical approaches have been used for interbody fusions. In 1956, Ralph Cloward developed a method and instrumentation for anterior spinal interbody fusion of the cervical spine. Cloward surgically removed the disc material and placed a tubular drill guide with a large foot plate and prongs over an alignment rod and then embedded the prongs into adjacent vertebrae. The drill guide served to maintain the alignment of the vertebrae and facilitated the reaming out of bone material adjacent the disc space. The reaming process created a bore to accommodate a bone dowel implant. The drill guide was thereafter removed following the reaming process to allow for the passage of the bone dowel which had an outer diameter significantly larger than the reamed bore and the inner diameter of the drill guide. The removal of the drill guide left the dowel insertion phase completely unprotected.

More recent techniques have advanced this concept and have provided further protection for sensitive tissue during disc space preparation and dowel insertion. Such techniques have been applied to an anterior approach to the lumbar spine. In one approach, a unilateral template has been provided to evaluate the space in the disc space. For bilateral implant placement, the template entire device must be rotated and visually aligned to approximately 180 from the previous position. Thus, there is the chance for operator error in rotating the device to the correct position. Further, there is little guidance to ensure proper alignment of cutting instruments extending through the template.

One approach to provide such alignment is the use of a guide wire extending through a cannulated cutting instrument, such as a trephine. However, for instruments with hollow cutting heads, there is typically no engagement between the inner walls of the hollow cutting head and the guide wire. Thus, the guide wire may bend between the portion extending into the tissue and the guide wire entrance into the cannula of the instrument. As a result, the hollow cutting head may not remain in substantial alignment with the guide wire, resulting in improper opening formation. Therefore, there remains a need for improved guiding mechanisms for cutting instruments.

Once an initial opening or openings have been made in the disc space, the height of the disc space is normally distracted to approximate the normal height. Typically, a first distractor with a height estimated by CT or MRI examination is inserted. If additional distraction is required, the first distractor is removed and a second, larger distractor is inserted. However, since the positioning of the distractors is usually performed without the benefit of protective guide sleeve assemblies, the switching of distractors increases the potential for damage to neurovascular structures and may increase the time of the procedure.

For bilateral procedures, a guide sleeve assembly may be inserted over a pair of previously placed distractors with a central distractor extending into the disc space to maintain distraction. One limitation on guide sleeve assembly placement is the amount of neurovascular retraction that must be achieved to place the guide sleeve assembly against the disc space. For some patients, a guide sleeve assembly may not be used because there is insufficient space to accept the guide sleeve assembly. Further, although the distal end of the guide sleeve assembly may be configured to engage the vertebral surface, if material has been removed from the disc space, there is the potential that adjacent neurovascular structures may encroach on the working channels in the disc space, resulting in damage to these structures caused by contact with instruments. While visualization windows on the guide sleeve assembly may assist in better visualization of procedure steps and verifying unobstructed working channels prior to tool insertion, the windows themselves may allow tissue to come into contact with instruments in the working channels. Thus, there remains a need for guide sleeve assemblies requiring reduced neurovascular retraction for proper placement and providing greater protection to adjacent tissue. WO-A-9627345 discloses apparatus for inserting spinal implants, the apparatus including a guide sleeve assembly with lateral extensions for maintaining vertebrae distracted in their normal angular relationship. The preamble of claim 1 is based on this disclosure.

With guide sleeve assemblies in place, the disc space and end plates may be prepared for receipt of an implant. Typically, cutting instruments are advanced to remove disc material and bone. Such operations may be time consuming since it is often necessary to adjust depth stop equipment and to remove the instruments to remove cutting debris. Since it is desirable to shorten the time of the operative procedure, there remains a need for improved cutting instruments and depth stop mechanisms.

While the above-described techniques are advances, improvement is still needed in the instruments and methods. The present invention is directed to this need and provides more effective instrumentation for achieving the same comprising a guide sleeve assembly according to the claims. The other instrumentation herein disclosed does not form part of the invention but is useful for understanding it.

The present invention relates to instrumentation for vertebral interbody fusion. The present invention provides a guide sleeve assembly, comprising:
a first tube defining a first inner working channel opening at a distal bone engaging end of said first tube and at least one lateral extension extending from said bone engaging end of said first tube having a first height;
a second tube defining a second inner working channel opening at a distal bone engaging end of said second tube and at least one lateral extension extending from said bone engaging end of said second tube having a second height, said first and second lateral extension inhibiting encroachment of tissue into an adjacent one of said inner working channels;
said first tube joined to said second tube to define said guide sleeve assembly having a longitudinal axis, said guide sleeve assembly having a proximal portion and an opposite distal portion adapted for insertion into a patient, said proximal portion having a first width and said distal portion having a second width transverse to said longitudinal axis; and
a distractor between said first and second lateral extensions said distractor defining a third height corresponding to a distracted disc space height when inserted into a spinal disc space, characterized by said second width being less than said first width and/or said third height being greater than said first and second heights. In one form the present invention may be used in a method which contemplates gaining access to at least a portion of the spine, marking the entrance point or points in the disc space, creating an initial opening in the disc space through a template, distracting the disc space and positioning a guide sleeve assembly defining an interior working channel adjacent the disc space. Preferably, the template can be inserted in a reduced sized configuration, with a first portion engaging the tissue. The template may then be manipulated to a larger configuration for bilateral insertion procedures by movement of a second portion, without repositioning the first portion. Additionally, a template may include trephine guides that accommodate a variety of different diameter trephine cutting heads. Specifically, trephines may include an upper shaft having a uniform diameter regardless of trephine cutting head diameter. Thus, the upper guides of the template maintain the trephine in axial alignment regardless of whether the lower guide engages the trephine head. The improved guide sleeve assembly is provided to maintain alignment of cutting instruments.

Once an initial opening or openings have been defined in the disc space, a distractor may be inserted to distract the disc space to the desired height. Various distractors may be used to distract the disc space. One such distractor has a first position that provides a first working distraction height in the disc space and a second position that provides a greater second working distraction height. Should the first working distraction height be insufficient, the distractor may be rotated one quarter turn to create a second greater distraction height in the disc space. Additionally, a modular distractor mechanism may be configured to accept many different rotatable distractor tips and may releasable engage the tips such that a distractor tip may be left in the disc space while permitting withdrawal of the distractor tool shaft. With such a configuration, a single distractor tool shaft may be use with various tips, thereby limiting the total number of complete distractor instruments required. Additionally, distractor tips may be made of radiolucent material that will not inhibit x-ray imaging of the disc space. Such distractor tips may include radiographic markers to indicate the ends and/or outer edges of the device and markers to indicate the rotational alignment of the distractors in the disc space.

Once the desired distraction of the disc space has been achieved, the handle of the distractor may be removed and a guide sleeve assembly positioned over the distractor. For a bilateral approach, one or both of the distractors may be left in position and a guide sleeve assembly positioned over the distractors and advanced toward the disc space. A further step that may be performed in a preferred embodiment is to select a removable distal tip for the guide sleeve assembly that matches the height of disc space distraction and the diameter of the implant. Thus, a guide sleeve assembly may be used with interchangeable distal tips to accomplish the insertion. Whether single (which would not be an embodiment of the present invention) or double, the guide sleeve assembly is advanced until the leading distractor portion of the guide sleeve assembly is adjacent the disc space. If necessary, a driving cap may be positioned over the proximal end of the guide sleeve assembly. The guide sleeve assembly is then driven into position, preferably with a spike or series of spikes engaging vertebrae adjacent the disc space.

Although various guide sleeve assemblies are known in the art, in some embodiments guide sleeve assemblies according to the present invention have a reduced width portion adjacent the distal end to limit the amount of retraction of the surrounding vascular and neural structures required for the procedure. In all other embodiments, a guide sleeve assembly includes a central distraction flange having a first height and an opposing pair of lateral extensions having a second height, less than the first height. The lateral extensions provide protection from encroachment of tissue into the working area in the disc space. A further aspect of a preferred embodiment includes the provision of visualization windows along the guide sleeve assembly for visual access to the interior working channel while instruments are in the working channel. Various combinations of windows are disclosed to accomplish the desired visualization. While visualization is desirable, having openings in the guide sleeve assembly may allow surrounding vessels and tissue to migrate into the working channel of the guide sleeve assembly. Tissue and vessels present in the working channel may be damaged by insertion and removal of the tools (often with cutting edges) or during use of those tools. Thus, the present invention contemplates covers over the windows that may be selectively opened for visualization and closed to prevent tissue and vessel infiltration. Additionally, the covers or the guide sleeve assembly may be transparent to allow visualization through the windows without removing the covers or directly through the guide sleeve assembly. In a similar manner, an image guidance system such as that available under the tradename STEALTH may be used in conjunction with the present system to monitor the advancement and positioning of instruments and implants. Even without the use of an imaging system, a manually adjustable depth stop may be used to control the steps of trephining, reaming, tapping, and dowel insertion. The term dowel is used in a broad sense throughout the disclosure and is intended to encompass dowels made of bone, metallic cages and other implants used for interbody fusion regardless of shape or material of construction.

One embodiment of the present invention comprises a guide sleeve assembly with a visualization window disposed adjacent a distal end and a cover removably covering the window. In one preferred embodiment, the cover includes a flange adjacent the distal end to mobilize vessels and other tissue away from the ends of the guide sleeve assembly. In one form, the cover is slidably disposed on the upper surface of the guide tube or tubes to cover only the upper windows. In another form, the cover is slidably positioned on the guide tube to cover the upper and lower windows of the guide tube.

The guide sleeve assembly according to the invention, has a double barrel configuration. The bone engagement end of the guide sleeve assembly includes a central distractor having a first height sufficient to maintain distraction. Preferably, the bone engaging end also includes a pair of opposing lateral extensions having a second height less than the first height. The lateral extensions are intended to inhibit lateral encroachment of tissue into the working area in the disc space but are not limited to maintain distraction.

An adjustable stop mounted on a tool shaft may be provided. The stop is selectively engageable with the tool shaft at a plurality of locations along the tool shaft by axial movement of a collar to control the position of the stop engaging portions. With the collar in a first position, the engaging portions are disengaged from the tool space. With the collar in the second position, the engaging portion is urged into engagement with the shaft. The tool shaft is sized to be received within a guide sleeve assembly and the stop is sized to prevent passage within the guide sleeve assembly. Thus, the stop may be selectively coupled to the tool shaft to control the extent of tool shaft that may be received within the guide sleeve assembly. Although not required, the stop may include a viewing window and the tool shaft includes markings, whereby the markings are calibrated to indicate to the user the extent of tool shaft extending beyond a distal end of the guide sleeve assembly.

A reamer may be provided with a reaming head having a plurality of reaming apertures in communication with an internal channel. The internal channel extends within the reaming head and proximally along at least a portion of the reamer shaft. The internal channel includes a proximal segment extending non-parallel to the longitudinal axis of the reamer shaft, whereby reaming debris may be transferred to the exterior of the reaming shaft.

A method for interbody fusion is envisaged comprising, positioning a template adjacent a fusion site, forming at least one initial opening in the disc space, distracting the disc space, placing a distal portion of a guide sleeve assembly in accordance with the present invention into the disc space, the guide sleeve assembly including at least one visualization window and cover removable disposed over the windows and visualizing the surgical site through the windows. Preferably, the method also includes removing the cover to expose the window prior to visualization. Further, the method may include the step of enlarging the opening with cutting tools and may further include attaching an adjustable depth stop to the tool shaft prior to extension beyond the distal end of the guide sleeve assembly.

There is also provided a template for marking a surgical site, comprising:
a first member having a first end and an opposite second end;
a second member having a first end and an opposite second end, said second member in circumferential engagement with at least a portion of said first member;
a first template member having a first configuration, said first template member connected to said first end of said first member; and
a second template member having a second configuration approximating to said first configuration, said second template member connected to said first end of said second member, wherein said second template member is moveable with respect to said first template member from a first position in substantial alignment with said first template member to a second position disposed approximately 180° in relation to said first position.

Preferably said second member includes a guide in substantial alignment with said second template, said first member first end further includes a post extension. Said first and second configurations may include substantially circular portions. The template may be provided in combination with a guide sleeve assembly, wherein in said second position, said first template member and said second template member define an area that approximates an area required for subsequent placement of the guide sleeve assembly adjacent the surgical site. The template may further include a locking mechanism selectively locking said first member to said second member.

Not forming part of the present invention, there is provided a guide sleeve assembly for a rotary cutting tool with a central opening, the guide sleeve assembly comprising:
a shaft having a distal end and a first diameter; and
an enlarged guiding portion disposed adjacent said distal end and adapted for engagement with the central opening of the cutting tool, said enlarged guiding portion having a second diameter, said second diameter greater than said first diameter. Preferably said enlarged guiding portion includes a tapered surface.

A distractor may be provided comprising:
a distraction head having a longitudinal axis and a first pair of opposed surfaces extending substantially along said longitudinal axis and defining a first working distraction height approximating a normal disc space height and a second pair of opposed surfaces extending substantially along said longitudinal axis and defining a second working distraction height approximating a second normal disc space height, said distraction head rotatable between said first distraction height and said second distraction height by rotating the distraction head about said longitudinal axis. Preferably said first working distraction height is 10mm and said second working distraction height 12mm. Alternatively said first working distraction height is 8mm and said second working distraction height is 10mm.

According to the invention a guide sleeve assembly for defining a protected passageway to a disc space is provided comprising:
a first guide tube defining a first working channel, and a first bone engaging end;
a second guide tube defining a second working channel and a second bone engaging end, said second guide tube connected to said first guide tube with said second bone engaging end disposed adjacent said first bone engaging end;
a central distractor disposed between said first working channel and said second working channel adjacent said first and second bone engaging ends, said central distractor having a first height;
a first lateral extension extending from said first bone engaging end opposite said central distractor, said first lateral extension having a second height; and
a second lateral extension extending from said second bone engaging end opposite said central distractor, said second lateral extension having a third height, said first height greater than said second height and said third height, wherein said central distractor maintains distraction in a disc space and said first and second lateral extensions inhibit encroachment of adjacent tissue into said first and second channels. Preferably each of said bone engaging ends includes spikes for engaging vertebral bodies.

It is preferred that said first guide tube is oriented substantially parallel to said second guide tube, and wherein the assembly has a proximal end opposite said bone engaging ends, said proximal end having a first width, said first bone engaging end and said second bone engaging end defining a second width, said first width being greater than said second width.

Said central distractor may have a thickness greater than a thickness of said first and second lateral extensions. Preferably said thickness of said central distractor is approximately twice a wall thickness of said first and second guide tubes.

There is additionally provided a guide sleeve assembly not according to the present invention, comprising:
a first barrel defining an inner working channel;
a second barrel defining an inner working channel;
said first barrel joined to said second barrel to define a guide sleeve assembly having a longitudinal axis, said guide sleeve assembly having a proximal portion and an opposite distal portion adapted for insertion into a patient, said proximal portion having a first width and said distal portion having a second width transverse to said longitudinal axis, said second width less than said first width. Preferably said first barrel and said second barrel each have a wall thickness, said wall thickness of said first and second barrel reduced adjacent said distal portion. Said first and second barrels may have a circular cross-section.

Not forming part of the present invention, there is provided a guide sleeve assembly for disc space preparation, comprising:
a guide tube defining an interior working channel, said guide tube having an outer surface and defining at least one window extending from said outer surface to said interior working channel, said window permitting visualization of the disc space; and
a cover slidably disposed on said outer surface adjacent said at least one window, said cover adapted to slide along an axis of said guide tube to selectively cover said at least one window. Preferably said cover includes a dovetail portion slidably disposed within a dovetail groove defined by said outer surface. Said cover may be slidable between a first window covering position and a second uncovered position. Said cover may be a partial cylinder. Preferably said partial cylinder extends approximately 200°. Alternatively said partial cylinder extends approximately 270°. Said cover preferably includes an outwardly extending flange, said flange adapted to retract tissue disposed adjacent said outer surface. Said guide tube may have a top side and an opposite bottom side, said guide tube having one of said at least one window on each of said top side and said bottom side. Preferably said cover selectively covers said one of said at least one window on said bottom side. Said cover may selectively cover said one of said at least one window on said top side. It is preferred that said cover selectively covers said one of said at least one window on each of said top and bottom sides.

Further, there is provided a reamer, comprising:
a reamer head having a plurality of cutting blades separated by a plurality of troughs, said reamer head defining an internal chamber and openings extending between said troughs and said internal chamber; and
a shaft joined to said reamer head, said shaft having a longitudinal axis and an exterior surface having an opening therein and defining an interior channel in communication with said opening, said interior channel in communication with said internal chamber, wherein debris entering said internal chamber during a cutting operation may exit via said interior channel in said shaft. Preferably said shaft includes a first diameter portion adjacent said reamer head and a second diameter portion spaced from said reamer head, said second diameter greater than said first diameter. Said interior channel preferably has a portion extending at an acute angle with respect to the longitudinal axis of said shaft.

There is yet further provided a depth stop for selectively engaging a tool shaft, comprising:
a first tubular member having a first end defining a plurality of flexible fingers moveable between a first position defining a first diameter and a second position defining a second diameter, said first diameter greater than said second diameter; and
a second tubular member disposed about said first tubular member, said second tubular member having a clamping end disposed adjacent said flexible fingers, said clamping end including an enlarged first internal diameter portion and a reduced internal diameter portion, said second tubular member slidable in relation to said first tubular member to a disengaged position with said enlarged internal diameter portion positioned adjacent said flexible fingers to allow movement of said flexible fingers to said first position and an engaged position with said reduced internal diameter portion positioned adjacent said flexible fingers to urge said flexible fingers to said second position. Preferably the tool shaft includes a series of annular grooves and said fingers include corresponding projections to engage the annular grooves. Said second tubular member may be biased to said engaged position.

There is still further provided a modular spinal cutting tool for removing a portion of vertebral bone or adjacent tissue, said tool comprising:
a shaft having a first connection end;
a cutting head having a shaft portion, said shaft portion including a second connection end; and
a connecting mechanism connecting said first connection end and said second connection end, said connecting mechanism including a collar slidably displaceable between an engaged position to connect said first and second connection ends and a disengaged position to disconnect said first and second connection ends. Preferably said cutting head is a reamer cutting head. Said cutting head may be a tap. Said collar may be slidable along an axis of said tool between said engaged position and said disengaged position. Preferably said connecting mechanism includes at least one ball, said at least one ball being disposed in an aperture defined by one of said first and second connection ends, said at least one ball being engaged within a groove defined in another of said first and second connection ends, when said collar is in said engaged position to connect said first and second connection ends, said at least one ball being released from said groove when said collar is in said disengaged position to disconnect said first and second connection ends. Said collar may have a reduced internal diameter to urge said at least one ball into engagement within said groove when said collar is in said engaged position, and an enlarged internal diameter to release said at least one ball from said groove when said collar is in said disengaged position. Preferably said tool includes a plurality of said balls and a corresponding plurality of said apertures and grooves.

There is also provided an expandable template for guided cutting, comprising a template body having a first portion defining a first opening and a second portion defining a second opening, said second portion movably coupled to said first portion and movable between a first position with said first opening in substantial alignment with said second opening and a second position with said opening spaced from said first opening.

There is described a method of disc space preparation, comprising:
providing an expandable bilateral template having a reduced insertion form and an expanded form for bilateral templating and trephine guiding;
accessing the spine;
inserting the template in the reduced insertion form;
positioning the template adjacent the disc space;
expanding the template;
inserting a trephine through the template to create a first opening and a spaced second opening in the disc space; and
removing the template and trephine. The method may further include inserting a first distractor in the first opening to distract the disc space to a first working height;
inserting a second distractor in the second opening to assist in distracting the disc space to the first working height; and
rotating the first and second distractors to create a second greater disc space height.

Related objects and advantaged of the present invention will be apparent from the following brief description of the drawings.
Fig. 1a is a perspective view of an expendable template
Fig. 1b is a side elevational view of the template of Fig. 1a.
Fig. 1c is front view of the template of Fig. 1a.
Fig. 1d is a top view of the template of Fig. 1a.
Fig. 1e is a bottom view of the template of Fig. 1a
Fig. 1f is an enlarged perspective view of the engaging end of the template of Fig. 1a.
Fig. 2a is a perspective view of the template of Fig. 1a in an expanded condition.
Fig. 2b is a bottom view of the template of Fig. 2a
Fig 3a is a side view of another embodiment of an expandable template with a trephine disposed therein.
Fig. 3b is a top view of the expandable template of Fig. 3a showing the locking mechanism.
Fig. 4a is a perspective view of a guide member and trephine
Fig. 4b is an enlarged perspective view of a portion of Fig. 4a.
Fig. 5a is a perspective view of a distractor
Fig. 5b is an enlarged front view of the tip of the distractor of Fig. 5a.
Fig. 5c is an enlarges side view of the tip of the distractor of Fig. 5a.
Fig. 6 is a perspective view of a guide sleeve assembly according to the present invention.
Fig. 7 is a from view of the guide sleeve assembly of Fig. 6.
Fig. 8 is a side view of the guide sleeve assembly of Fig. 6.
Fig. 9 is a partial cross-sectional side view of a guide sleeve assembly with a removable tip.
Fig. 10 is a perspective view of a guide sleeve assembly with a cover according to the present invention.
Fig. 11 is an end view of the guide sleeve assembly of Fig. 10.
Fig. 12 is a front view of one embodiment of a guide sleeve assembly window cover according to the present invention.
Fig. 13 is a front view of a guide sleeve assembly with the cover of Fig. 12 mounted thereon.
Fig. 14 is a perspective view of an engaging end of a guide sleeve assembly with another embodiment of a window cover according to the present invention.
Fig. 15a is a side view of a window cover.
Fig. 15b is an end view of the window cover of Fig. 15a.
Fig. 16a is still a further embodiment of a window cover in accordance with the present invention.
Fig. 16b is an end view of the window cover of Fig. 16a.
Fig. 17 is an anterior to posterior view of a guide sleeve assembly with window covers according to Fig. 15 disposed thereon, the guide sleeve assembly is positioned in relation to a pair of adjacent vertebral bodies and blood vessels.
Fig. 18 is a partial cross-sectional top view of a guide sleeve assembly with only one window cover positioned thereon, a portion of the guide sleeve assembly extending into the disc space.
Fig. 19 is a side view of a hollow headed reamer
Fig. 20 is the reamer of Fig. 19 rotated 90 degrees about the shaft longitudinal axis.
Fig. 21 is an enlarged partial cross-sectional view of the head of the reamer of Fig. 19.
Fig. 22 is a side view of a clean out tool for use with the hollow reamer head of Fig. 19.
Fig. 23 is a top view of the clean out tool of Fig. 22.
Fig. 24 is a side view of a tap
Fig. 25 is a side view of a tap having a removable tap head
Fig. 26a is a side view of a reamer having a removable reamer head
Fig. 26b is a partial cross-sectional view of the connection mechanism of Fig. 26a.
Fig. 27 is a perspective view of a depth stop with the collar partially retracted to expose the locking fingers.
Fig. 28 is a side view of the depth stop of Fig. 27.
Fig. 29 is a cross sectional view taken along line 29-29 of Fig. 28.
Fig. 30 is a front view of the depth stop of Fig. 27 with the collar fully extended.
Fig. 31 is a side view of an alternative embodiment of a depth stop
Fig. 32 is a partial side view illustrating the depth stop of Fig. 31 in engagement in with a tool shaft.

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as fall within the scope of the claims.

The present invention relates to instrumentation for performing vertebral interbody fusion. The instruments disclosed herein are particularly useful for anterior lumbar interbody fusion.

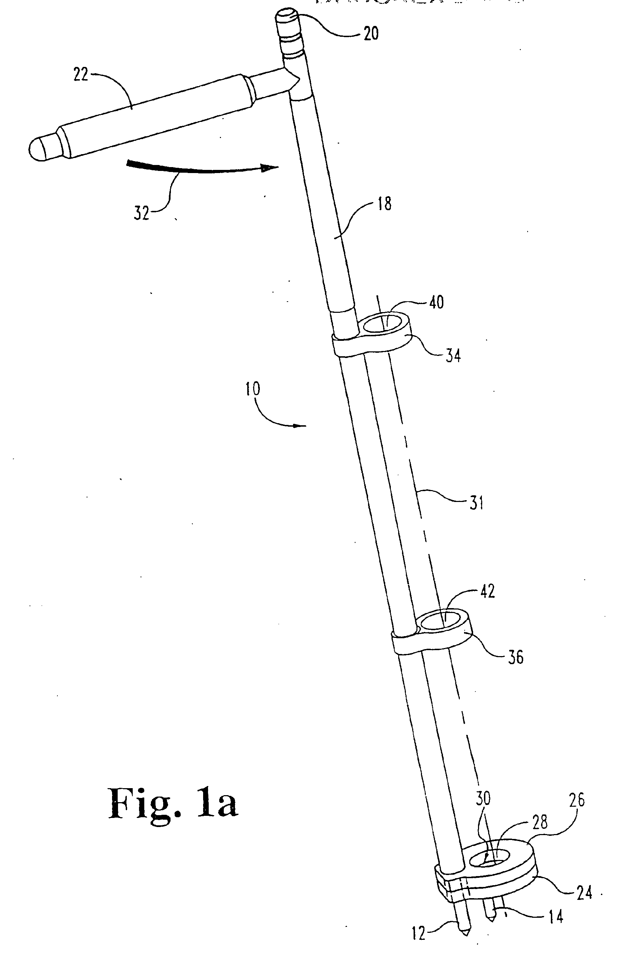

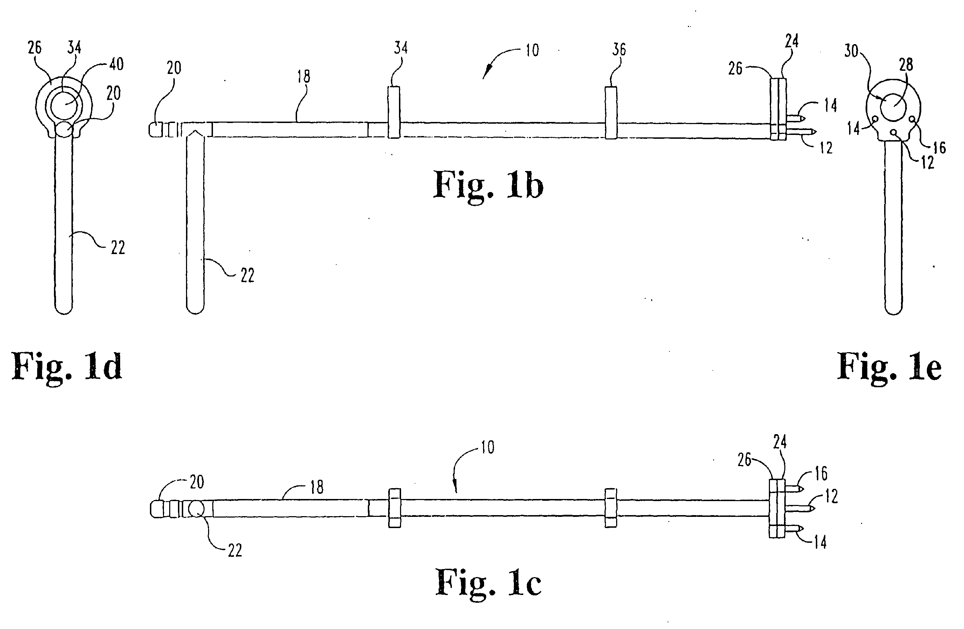

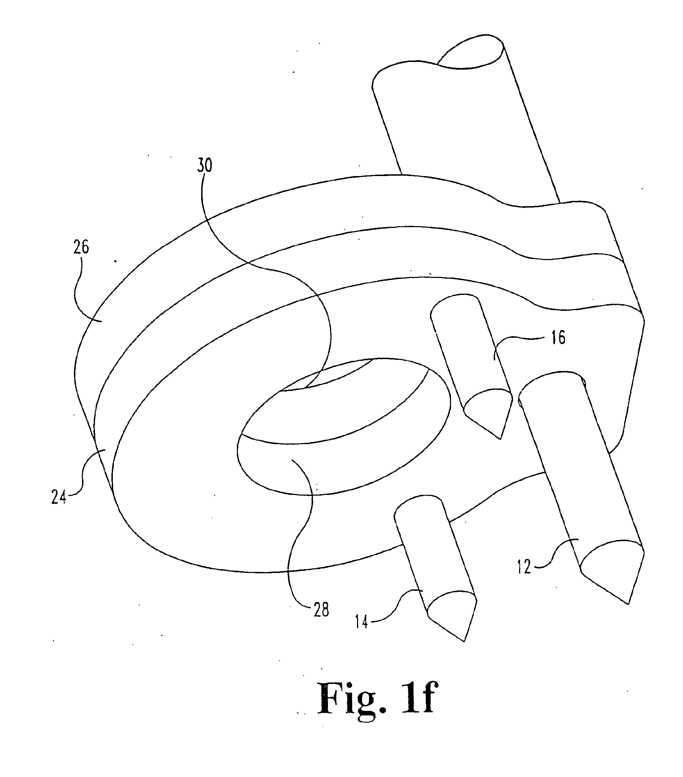

Referring now to Figs. 1(a) through (f), there is shown an intraoperative template 10 for use in interbody fusion. Intraoperative template 10 includes a central anchoring pin 12 and two supplemental anchoring pins 14 and 16. These pins are adapted to be driven into vertebral bodies or other tissue adjacent a disc space to anchor the intraoperative template 10 in the proper location. Template 10 includes an outer shaft 18 interconnected with handle 22 and an inner shaft 20 disposed within outer shaft 18. Inner shaft 20 extends to encompass pin 12. Outer shaft 18 is rotatable with respect to inner shaft 20. Disposed adjacent the distal end of template 10 are guide members 24 and 26 connected to inner shaft 20 and outer shaft 18, respectively. Preferably, guide members 24 and 26 are substantially circular plates having an aperture therein. Guide members 24 and 26 define openings 28 and 30, respectively, adapted to receive a trephine tool therethrough. Trephine guides 34 and 36 are positioned along outer shaft 18 and have openings 40 and 42, respectively, in alignment along axis 31 and are sized to receive a trephine tool shaft. In an alternative embodiment, it is contemplated that inner shaft 20 may be connected to guide member 26 and outer shaft 18 may be connected to guide member 24.

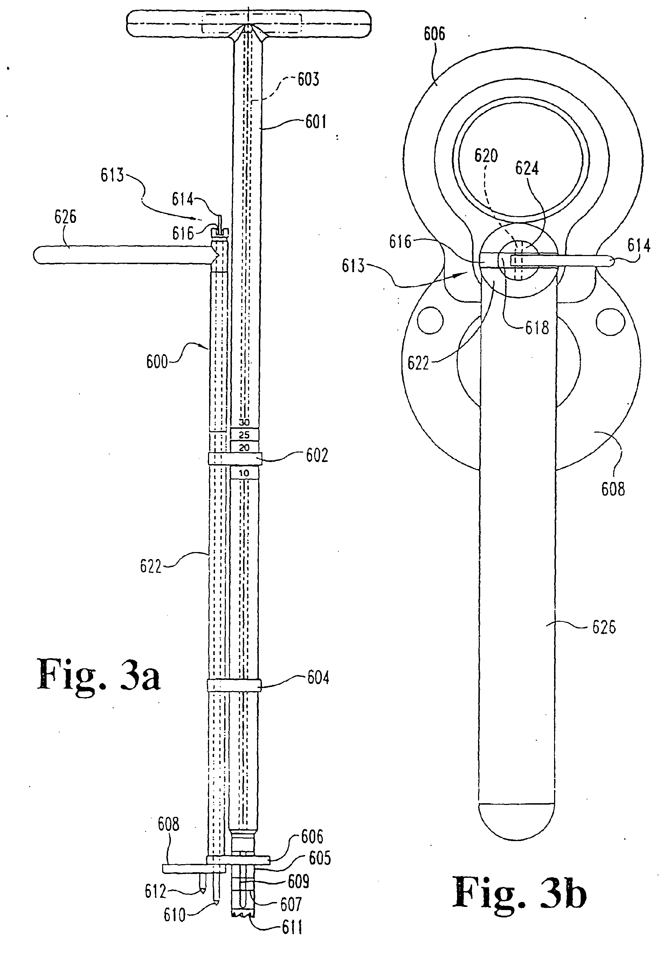

In a first reduced size configuration for unilateral templating and guiding, shown in Fig. 1a, guide members 24 and 26 are axially aligned along axis 31 with openings 28 and 30, respectively, m similar alignment. In this reduced size configuration, the expandable template may be inserted into the body through a relatively small opening and the template may be used for unilateral templating and guiding of a trephine. In this position, a trephine may be guided through guides 34 and 36 and guide members 24 and 26 to engage the tissue below. Moreover, referring to Fig. 3a, a trephine may have a uniform diameter along most of its shaft such that it is a dose fit within guides 34 and 36. The close fit in guides 34 and 36 maintains axial alignment, while permitting trephine shaft rotation. Thus, a single template 10 may be used with a variety of sizes of trephine head diameters, provided the shaft has a substantially uniform diameter.

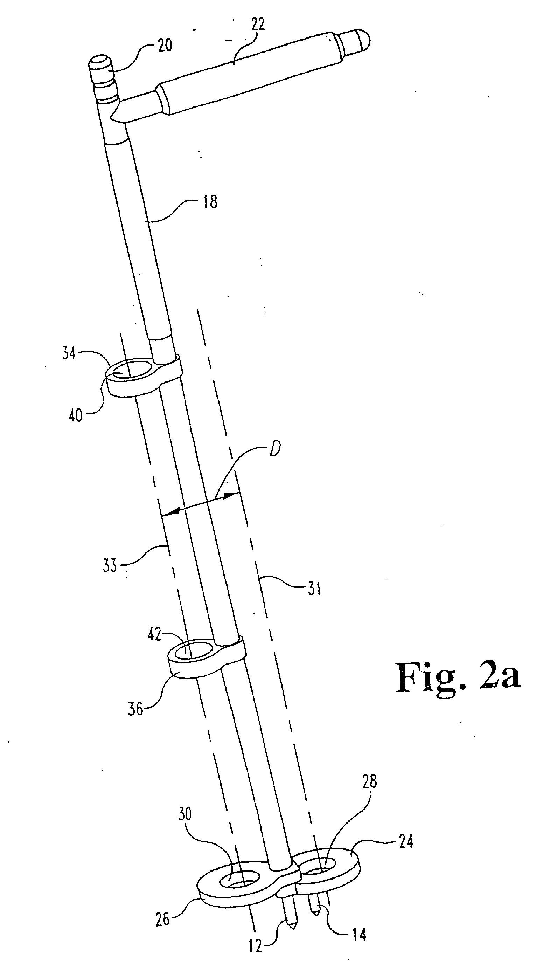

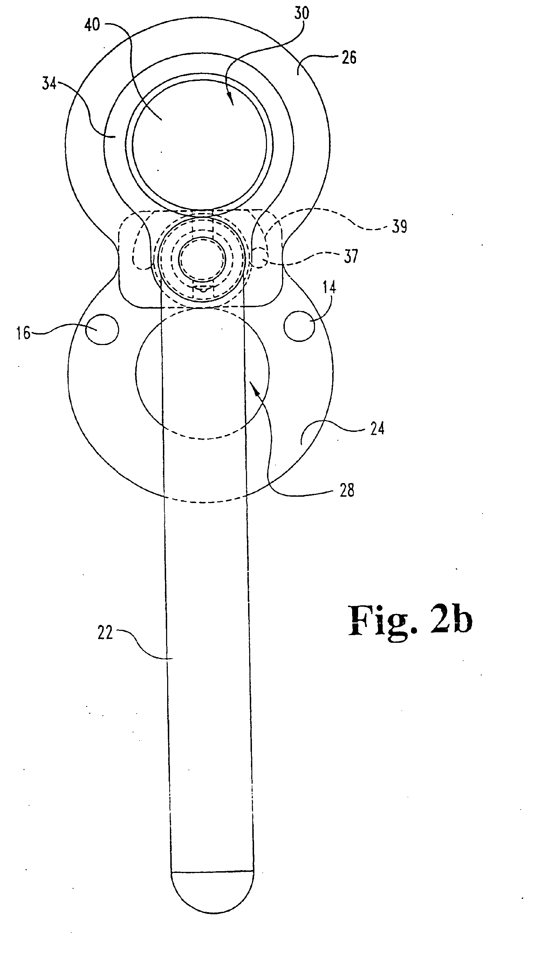

Referring now to Fig. 1a, handle 22 is connected to outer tube 18 and may be rotated in the direction of arrow 32 to a bilateral templating and guiding position. This action rotates outer shaft 18 with respect to inner shaft 20. Guiding member 26, guide 34 and guide 36 are connected to outer shaft 18 and therefore rotate when handle 22 is moved. In contrast, first guide member 24 is interconnected with inner shaft 20 and remains stationary upon rotation of handle 22. As shown in Fig. 2a, handle 22 is rotated approximately 180 degrees to align second template 26 approximately 180 degrees from first template 24 and thereby expand the template to its bilateral trephining position. Thus, a trephine procedure may be conducted along axis 33 through guides 34 and 36 and second member 26 to cut an opening in the disc space. Axis 33 is spaced from axis 31 by a distance "D" representing the distance of spacing of the midpoints between implants to be inserted. Figs. 2a and 2b show the first and second templates rotated 180 degrees with respect to one another. Fig 2b shows a bottom view of a bilateral templating and guiding configuration. In this expanded configuration, the outer edges of guide members 24 and 26 define the total area necessary for placement of implants and instruments having a specific configuration and size. While in a preferred embodiment, cylindrical implants having diameters of 16mm, 18mm or 20mm may be used, it is contemplated that other diameters may be used and other shapes such as, but without limitation, squares and rectangles.

Shown in dashed line in Fig. 2b is a groove 39 formed in guide member 24 and projection 37 defined on guide member 26 and extending into groove 39. It will be understood that the engagement between groove 39 and projection 37 maintains alignment and limits rotation to 180 degrees. Thus, template 10 may be moved between the reduced size configuration and expanded configuration, but the groove and projection engagement limit further movement and will provide a positive indication of 180° rotation, thereby eliminating the requirement for visual alignment with the first position.

Referring now to Fig. 3a, there is illustrated a further embodiment of an expandable template. Template 600 is substantially identical to template 10 previously disclosed above, with the exception that template 600 includes a locking mechanism 613. Expandable template 600 includes a handle 626 connected to outer shaft 622. As in the previous embodiment, template 600 includes a first guide member connected to inner shaft 624 and a second guide member 606 connected to outer shaft 622. First guide member 608 includes spike 612 and inner shaft 624 extends to form central spike 610. Outer shaft includes guides 602 and 604. As shown in Fig. 3a, a trephine 601 may be positioned through guides 602 and 604, and through guide member 606. The cutting head 605 includes cutting teeth 611, a series of index markings 607 and a window 609 to visualize the contents in the hollow interior. Preferably, trephine 601 includes a central cannula 603 extending from the handle to the cutting head.

A locking mechanism 613 is disposed between the inner and outer shafts to prevent rotation. Referring to Fig. 3b, locking arm 614 is pivotally attached to inner shaft 624 by pivot pin 620. The locking arm may be pivoted to extend through slot 616 in the outer shaft and slot 618 in the inner shaft. It will be understood that with locking arm disposed in the slots the inner and outer shaft will be prevented from rotation. In a first locked position, the shafts are aligned as shown in Fig. 1a in the reduced size configuration. In a second locked position, the shafts are aligned as shown in Fig. 3a in the expanded bilateral templating configuration. It will be understood that the expandable, rotatable template permits insertion of the device through a smaller opening than would have been permitted with a fixed relation double trephine opening template. Further, the expandable template may be locked in either a unilateral or a bilateral position. Locking engagement in the bilateral position insures accurate bilateral placement with consistency that would not be readily achievable with a unilateral template particularly where the surgeon must reposition the device by visual alignment. Subsequently, the device may be rotated to an expanded configuration suitable for trephine guiding to form bilateral openings without removing the instrument.

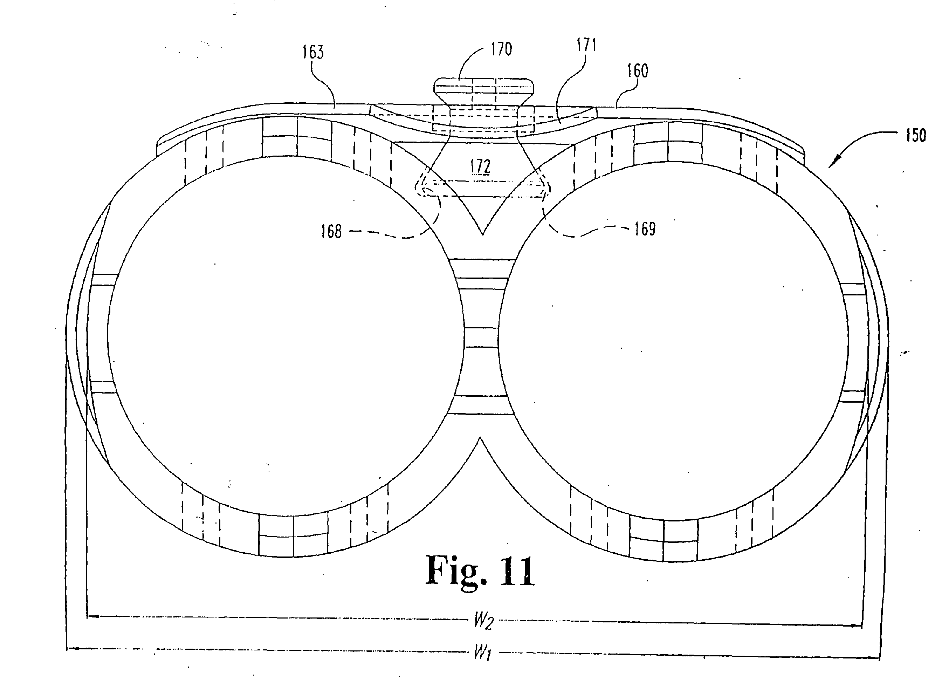

In use, access to an anterior portion of the spinal column is achieved by known method. Blood vessels, particularly the aorta, vena cava. and branches thereof are mobilized to provide space for bilateral implant placement. With the template in the reduced size configuration of Fig. 1a, the template is inserted into the body and advanced until the pins are disposed adjacent a disc space. The circumference of the template guide member is selected to the circumference needed for bilateral placement of a pair of implants. More specifically, the area of the guide members of Fig. 2b closely approximates the area needed for placement of the guide sleeve assembly disclosed herein, see for example Fig. 11. Central pin 12 is disposed centrally between the intended location of the implants. In either the unilateral or expanded bilateral condition, the template may be disposed adjacent the disc space to measure the space available for implant and instrument placement. If the space appears too small, a smaller sized template may be inserted to evaluate the space. In the bilateral condition, the template approximates the area needed for implant and instrument placement. Vessels disposed within the templated area may need to be mobilized outside the area or an alternative implant size or approach may be utilized. Further, osteophytes that appear within the templated area may be removed to prepare for engagement with a guide sleeve assembly. Once the area is cleared, the pins are inserted into the tissue of the disc space and/or adjacent vertebra to anchor the template, thereby maintaining its position during subsequent steps. As shown in Fig. 3a, a trephine is inserted into the guides and through the guiding members. The trephine is cuttingly advanced into the disc tissue to form an opening therein. The trephine may then be at least partially removed from the template to permit movement between the first and second guide members. If a lock mechanism is used, the locking arm must be moved to an unlocked position and the handle rotated to rotate the upper guide member to the expanded bilateral templating position. The trephine is reinserted and advanced through the upper guide member to form a second opening aligned with and offset a distance D from the first opening. Thus, the template permits controlled bilateral opening formation through an expandable and collapsible template. The template may be collapsed into its reduced size form and withdrawn after completion of the trephining operation.

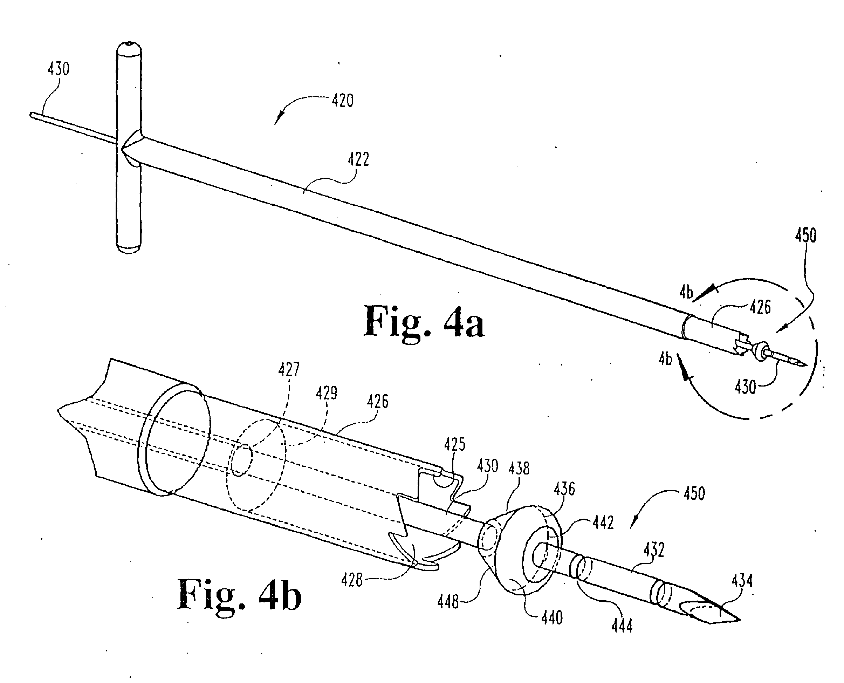

Referring now to Figs. 4a and 4b, there is shown a further guiding device not forming part of the invention. Guide sleeve assembly 450 includes an elongated shaft 430 having a substantially uniform diameter over most of its length. Shaft 430 includes a distal portion adapted for guiding a cutting instrument having a hollow cutting head. The distal portion of shaft 430 includes distal end 432 having a sharpened tip 434 adapted to penetrate tissue, specifically tissue disposed in the disc space. Distal end 432 includes markings 444 which indicate the extent of shaft 430 disposed in the disc space. Although the guide sleeve assembly 450 is preferably formed of stainless steel, other bio-compatible materials are contemplated. Specifically, shaft 430 may be formed of a radiolucent material and markings 444 may be radiopaque. Adjacent distal end 432 is enlarged portion 436 having a diameter substantially greater than the shaft diameter. Enlarged portion 436 is adapted to prevent further advancement of guide sleeve assembly 450 into the tissue and to guide the cutting of the cutting tool. Enlarged portion 436 preferably includes a planar surface 442 substantially perpendicular to the longitudinal axis of shaft 430. A substantially spherical surface 440 is disposed adjacent planar surface 442. This is followed by a tapering conical surface 438 that is adapted to align the cutting head over enlarged end 436. It will be understood that the internal surface of cutting head 426 defining opening 428 engages the transition line 448 between spherical surface 440 and taper surface 438. The diameter of transition line 448 substantially matches the internal diameter of cutting head 426 to provide a close fit for maintaining alignment.

In use, guide sleeve assembly 450 is inserted into the body with distal end 432 fully inserted into the tissue of interest, preferably disc tissue although other uses are contemplated. Cutting tool 420 is advanced over guide sleeve assembly 450 with shaft 422 in substantial alignment with shaft 430 extending through channel 427. While a trephine is illustrated, other cutting tools such as, but without limitation, reamers and non-rotary cutting tools may be used with guide sleeve assemblies.

Cutting teeth 425 are positioned adjacent enlarged portion 436 and are advanced until the cutting teeth surround the enlarged portion. It will be understood that if cutting teeth are offset with respect to enlarged portion 436, the teeth will engage a portion of conical surface 438 and thereby be urged into alignment. Enlarged portion 436 is received within chamber 428 and cutting teeth 425 are advanced along distal portion 432 until conical surface 428 abuts internal conical surface 429 to prevent further advancement. The guide sleeve assembly 450 may be withdrawn with the cut tissue impaled by distal portion 432. The tissue may be removed from chamber 428 by advancing the guide sleeve assembly 450 with respect to the cutting head such that the enlarged portion urges the tissue out of the hollow interior. This may be particularly helpful where the cutting tool is used to extract a bone graft. The depth of cutting teeth penetration may be adjusted by placement of the enlarged portion. Additionally, while only a single enlarged portion is shown, more than one may be positioned on the shaft to further adjust the guide sleeve assembly depth and cutting depth of the tool.

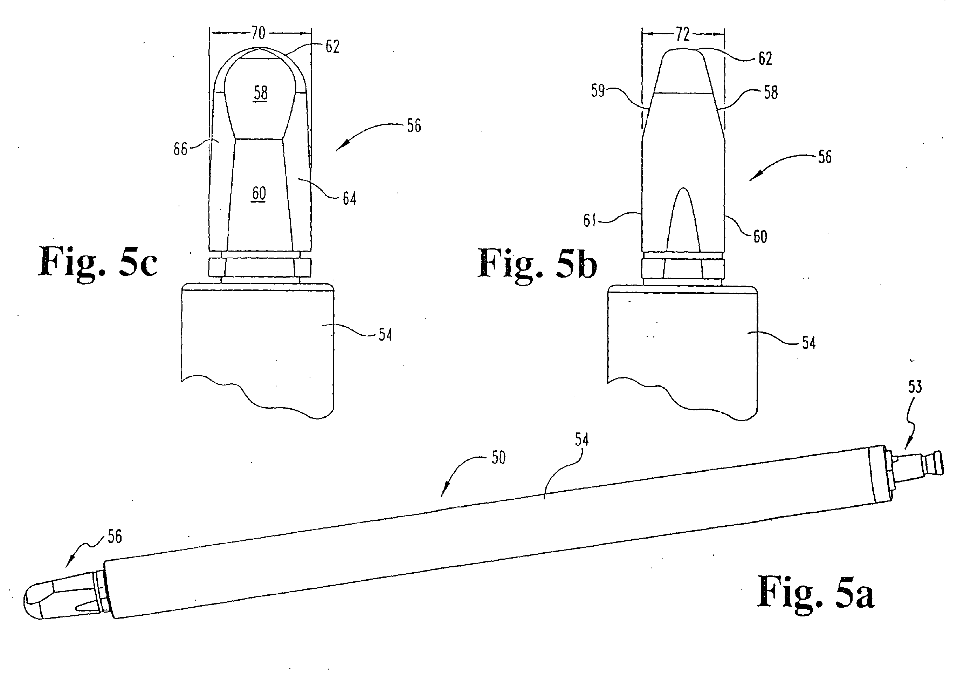

Referring now to Figs. 5a-c, there is shown a disc space distracter 50 . Distractor 50 includes a proximal end 53 configured as an enlarged end for engagement with a conventional Hudson connection on a T-handle (not shown). Shaft 54 is joined with a distracter tip 56. While an integral shaft and head are shown, head 56 may be removably attached to shaft 54. One such removable attachment is more fully disclosed in US 6,428,541. Distracter tip 56 is designed such that it can be inserted in a disc space to establish a first working distraction height 72 (see Fig. 5b), which is less than a second working distraction height 70 (see Fig. 5c). More specifically, distracter tip 56 has a rounded leading edge 62 that extends to opposing inclined surfaces 58 and 59 which extending more proximally blend into substantially planar opposing surfaces 60 and 61, respectively. Planar surfaces 60 and 61 extend in parallel alignment along the longitudinal axis of the distracter to establish height 72. It will be understood that the inclined surfaces 58 and 59 cooperate to ease insertion into the disc space and to initially distract the disc space to at least a height 72. If first height 72 is sufficient, further procedures as known in the art may then be carried out to accomplish implant insertion. Alternatively, rounded leading edge 62 permits the distractor to be inserted to directly achieve second distraction height 70.

Should first height 72 be insufficient, head 56 may be rotated a quarter turn, or 90 degrees, to the position shown in Fig. 5c. Rounded surfaces 64 and 66 engage the bone to urge it apart and into a second larger distracted height 70. It will be understood that utilization of a distracter tip as disclosed permits a two-height distraction of the disc space that may be carried out with a single instrument and without removing the instrument from the disc space. This offers an advantage to the surgeon of a single instrument offering multiple useful distraction heights. Thus, a surgeon may initially believe a disc space will need a first amount of distraction. After insertion of the distractor, the surgeon may discover that further distraction is required. In this situation, a distractor allows further disctraction without instrument withdrawal. Moreover, distractor head 56 limits the number of instruments that must be made available to surgeon to accomplish a surgical procedure by providing two working distraction heights on a single tool. Specifically, but without limitation, the distraction heads may be formed with first heights 72 ranging from 6mm to 12mm and second heights ranging from 7mm to 13mm. Preferably, heights 70 and 72 vary by 2mm increments. More preferably, height 72 is 8mm and height 70 is 10mm. In another form, height 72 is 10mm and height 70 is 12mm. Other variations may be utilized that provide multiple working distraction heights that approximate the disc height in a normal spine.

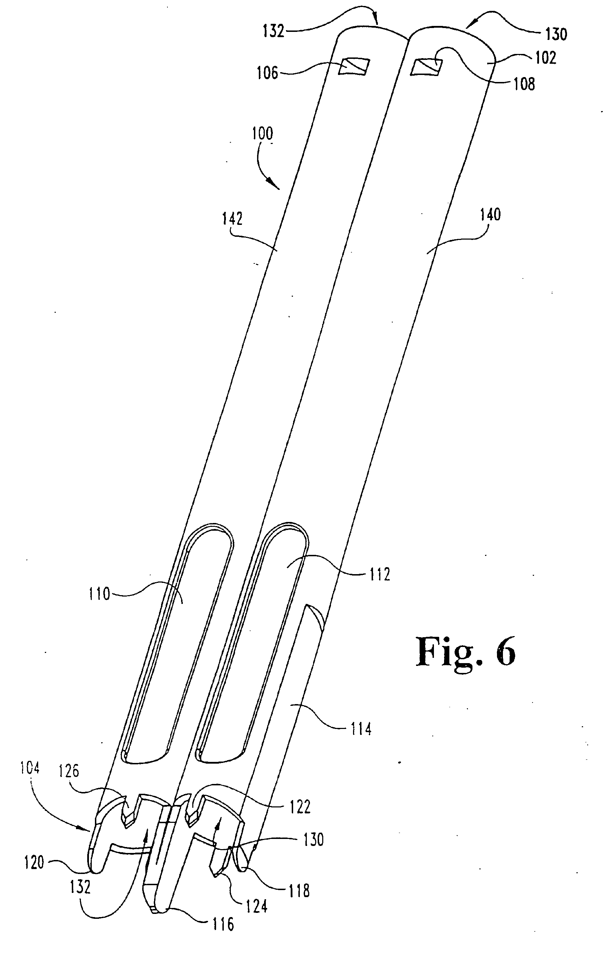

Referring now to Fig. 6, there is shown a double-barrel guide sleeve assembly in accordance with the present invention 100 having a first guide tube 140 connected to a second guide tube 142. Guide tubes 140 and 142 each define working channels 130 and 132 extending in a substantially unobstructed manner from the proximal end 102 to distal end 104. Assembly 100 includes upper windows 106 and 108 formed in guide tubes 142 and 140, respectively, that are adapted for engagement by a removal tool. The guide tubes also include lower elongated visualization windows 110 and 112.

Adjacent distal end 104, the material thickness along the outer edge of each guide tube 140 and 142 is reduced in order to provide a smaller cross-sectional area for the guide sleeve assembly as well as a reduced width extending transverse to the longitudinal axis of the guide sleeve assembly. The reduced cross-sectional area and smaller width reduces the amount of retraction of vessels adjacent the disc space that would be required without the reduction. Side wall 114 is shown as an indication of the reduced thickness of the guide sleeve assembly in the distal area 104.

Distal end 104 includes a central distractor 116 which may be inserted into the disc space to achieve or maintain a height H1 of distraction between two vertebral bodies. Lateral extensions 118 and 120 also extend partially into or adjacent to the disc space. However, in a preferred embodiment, lateral extensions 118 and 120 have a height H2 that is less than height H1. Thus, they do not provide distraction of the disc space but are provided primarily to protect surrounding vessels and neurological structures from damage during the procedures. Although that is the function of the lateral extensions in the preferred embodiment, it is contemplated that they could be sized to provide distraction within the disc space in conjunction with central distractor 116. Additionally, distal end 104 includes spikes 122, 124, 126, and a fourth spike which is not seen in the view of Fig. 6. These spikes may be urged into the bone of the adjacent vertebral bodies to hold the double-barrel guide sleeve assembly 100 in a fixed position relative to the vertebral bodies. It will be understood that windows 110 and 112 provide the medical staff with the opportunity to visualize the instruments as well as the openings in the disc space and vertebral bodies, without entirely removing instrumentation from guide sleeve assembly 100.

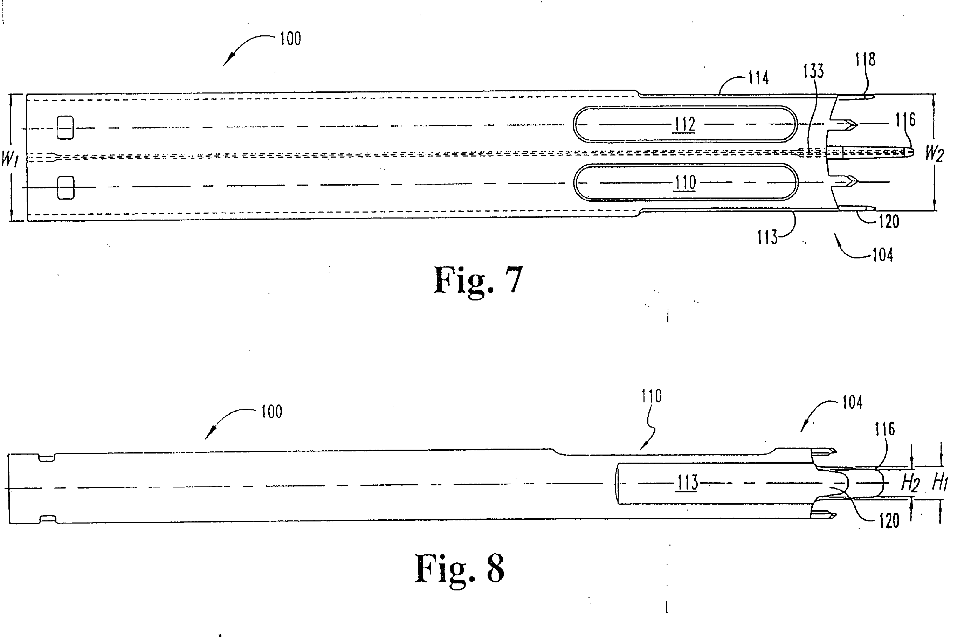

Referring more specifically to Fig. 7, double-barrel guide sleeve assembly 100 is shown in a front view to further illustrate an additional aspect of the invention. Opposite vertebrae engaging end 104, the guide sleeve assembly has a width W1 approximately twice the diameter of one of the guide tubes. Adjacent vertebrae engaging end 104 of the guide sleeve assembly, each of the outer portions of the guide tubes has a reduced wall thickness at side walls 114 and 113. The walls are not entirely flat but have a substantially greater radius of curvature (see Fig. 11) giving the appearance of substantially flat walls but providing a reduction in wall thickness over a greater area and tapering to the full wall thickness at the termination of side walls 113 and 114. The reduced wall thickness on the lateral portion of each guide tube reduces the overall width of the guide sleeve assembly to a width W2. The reduction in width decreases the amount of retraction that vessels in the area must be moved. The desirable reduction in width is accomplished with little reduction in the strength of the guide sleeve assembly since much of the structural integrity, particularly resistance to axial compression during insertion of the guide sleeve assembly, is carried by the much thicker central portion where the two guide tubes are joined to each other. Preferably, the central portion may have a thickness equal to two tube wall thickness.

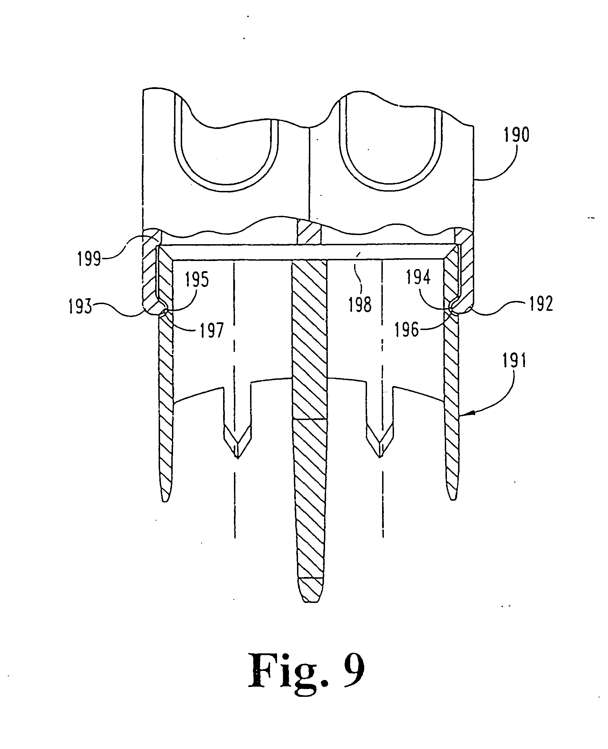

As a further alternative, Fig. 9 shows guide that guide sleeve assembly 190 may be provided with removable barrel tips 191 having different distraction heights, lateral extensions, or spike patterns. Barrel tips may also have different diameters corresponding to the placement of implants with different diameters. Removable tips 191 may be held in place by any of a variety of known connection mechanisms. However, in a preferred embodiment, guide sleeve assembly 190 includes a pair of opposing flexible fingers 192 and 193 having projections 194 and 195, respectively. Projections 192 and 193 on the flexible fingers extend into grooves 196 and 197, respectively, defined in the removable tip. To limit proximal movement of tip 191 during insertion, tapered surface 198 abuttingly engages shoulder 199 and the central portion between the upper guide tubes. Use of a removable tip according to the present invention not only allows use of interchangeable tips to suit a specific application, it also permits removal of the guide sleeve assembly after placement in the body. With only tip 101 in place, the posterior aspect of the disc space or spinal canal may be more easily visualized and accessed.

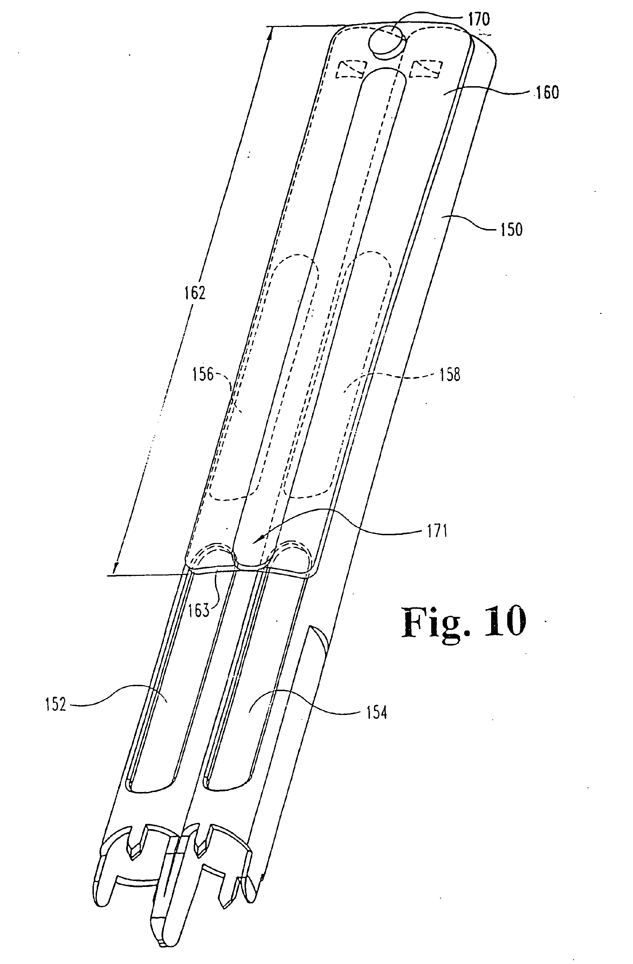

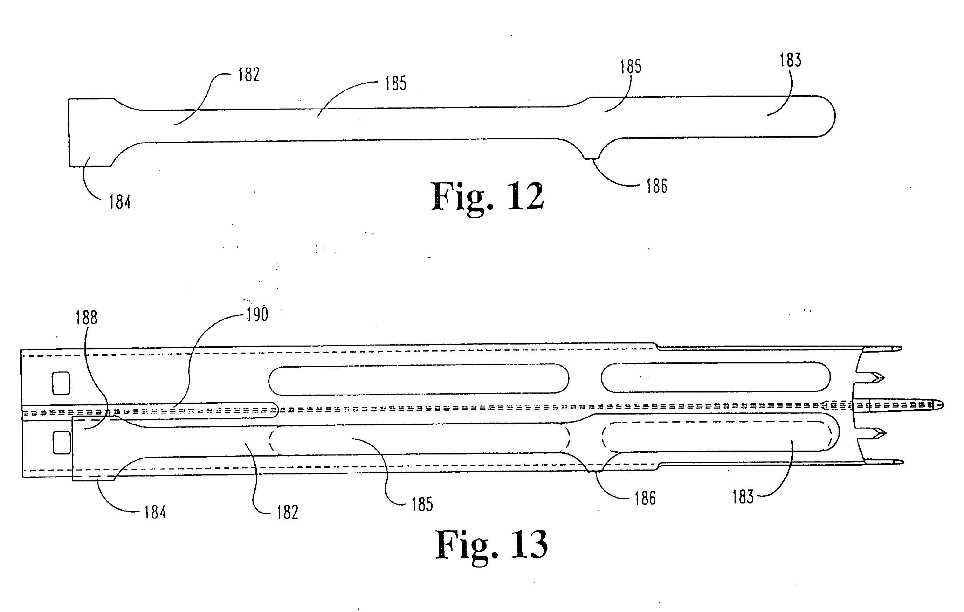

Referring now to Figs. 10 and 11, there is shown a further embodiment of a double-barrel guide sleeve assembly similar in most respects to guide sleeve assembly 100 of Fig. 6. The further embodiment of Fig. 12 differs from that of Fig. 6 in that guide sleeve assembly 100 included only a single elongated visualization window for each guide tube. In double-barrel guide sleeve assembly 150, each guide tube has a total of four windows, two on an upper surface and two on a lower surface. Thus, as shown in Fig. 10, windows 152, 154, 156, 158 provide the surgeon with the opportunity for visualization along the majority of each working channel. The back side of guide sleeve assembly 150 has a similar configuration.

Guide sleeve assembly 150 is used in a similar fashion to the guide sleeve assembly 100. In a preferred embodiment, guide sleeve assembly 100 is provided with a cover 160 having a length 162 sufficient to cover all four windows disposed on at least one side of the guide sleeve assembly. Cover 160 is provided to prevent possible damage to tissues which may invade the working channel through the windows and be damaged by the operation, insertion or removal of tools in the working channels. It is contemplated that cover 160 may be transparent to allow visualization directly through the cover or that it could be opaque, requiring that the cover be repositioned prior to visualization. It is further contemplated that the cover may have a length 162 sufficient to extend over all the windows on one side and it may be able to selectively cover either proximal windows 156 and 158 or all of the windows. Leading edge 163 is tapered to prevent damage to tissue, particularly when moving forward to cover the windows. The taper should urge the tissue out and away from the guide sleeve assembly. Further, cover 160 includes a dip 171 substantially following the contour between the pair of guide tubes.

Although other attachment mechanisms are contemplated, as shown in Fig. 11, cover 160 is held in place by retaining pin 170 connected through cover 160 to a lower dovetail portion 172. Dovetail portion 172 is slidable along a dovetail groove defined by grooves 168 and 169 defined within the outer body of guide sleeve assembly 150.

Figs. 10 and 11 show one embodiment of a cover for slidably and selectively covering a plurality of windows in guide sleeve assembly 150. Figs. 12 through 16b illustrate yet further embodiments of a cover which may be displaced to expose underlying windows in one of the guide tubes. Further, although the covers are disclosed for use with double barrel guide sleeve assemblies, it is contemplated that they may be used with single barrel guide sleeve assemblies not forming part of the invention without undue modification. In the further embodiments, the working channel and visualization windows of one barrel may be exposed while a cover remains in place on the alternate barrel.

Referring to Fig. 12, partially cylindrical cover 182 consists of elongated portions 183 and 185 which are sized to cover underlying visualization windows. The elongated portions are retained on the guide sleeve assembly by connectors 184 and 186 that are sized to extend around the exterior of the outer tube and guiding portion 188. It is contemplated that connectors 184 and 186 may engage a cover portion on the oppostie side of the guide sleeve assembly identical to that shown in Fig. 12. While cover 182 is disclosed as having elongated members 183 and 185 interconnected, it is contemplated that each of the covers 183 and 185 could be separate to allow visualization of the windows only on an upper or lower surface of the working guide tube without opening the opposing window.

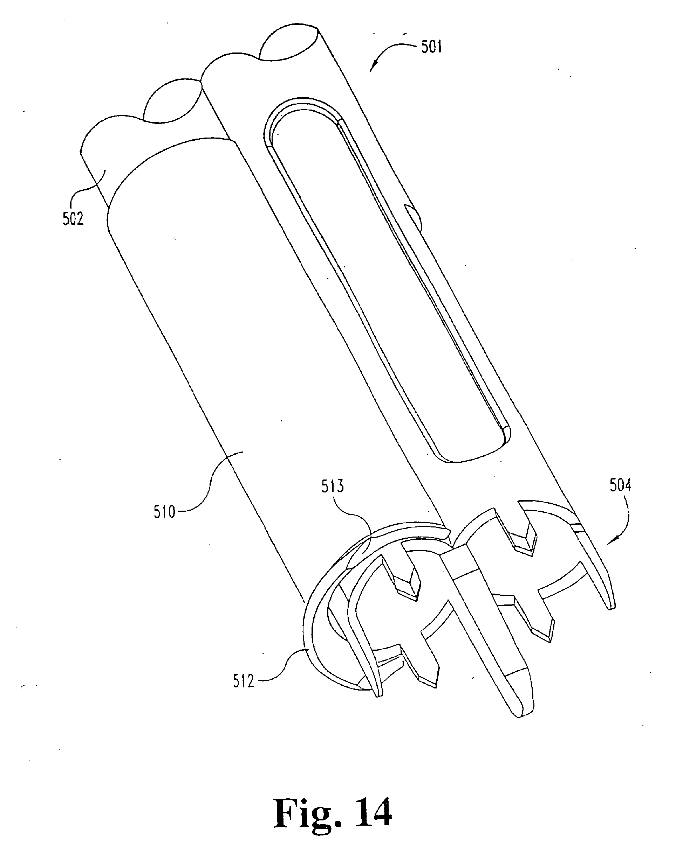

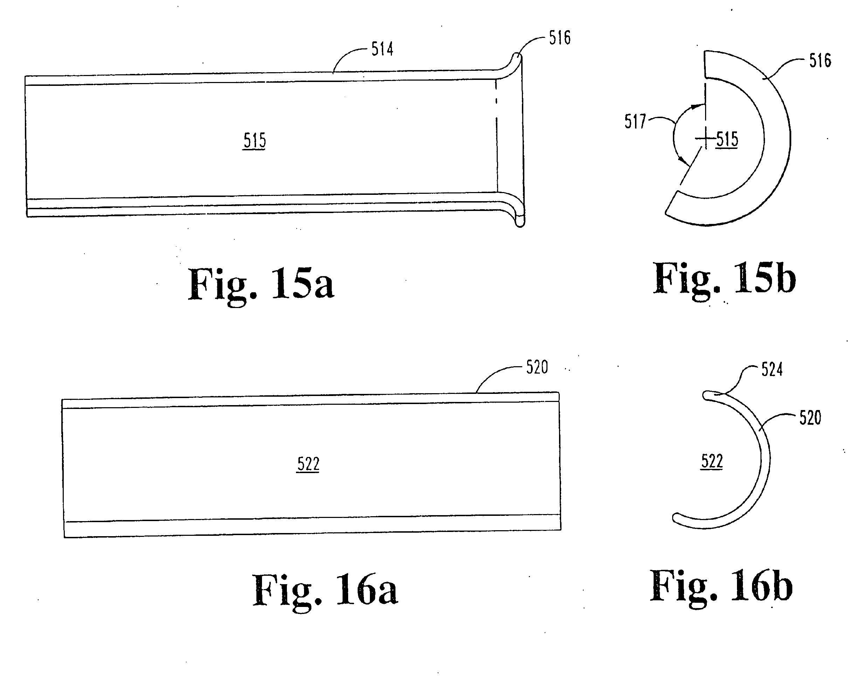

Referring to Figs. 14 through 16b, there are shown still further embodiments of window covers according to the present invention. Fig. 14 shows a cover 510 that covers approximately 200° of a single guide tube 502 of a guide sleeve assembly 501 similar to that of Fig. 6. The cover includes an internal passage 515 and is slidable along guide tube 502. In a further aspect, cover 510 includes an enlarged flange 512 adjacent bone engaging end 504. Tapered surface 513 extends between flange 512 and the outer diameter of cover 510. Referring to Figs.15a and 15b, cover 514 includes a flange 516 that extends along the entire leading edge of the cover. The cover extends in a partial cylinder lacking material over angle 517.

Angle 517 is approximately 160°, thus material extends around approximately 200° of the cylindrical shape. It will be understood that covers 510, 514, and 520 may be configured to have material extending less than 200° around the cylinder to allow rotation of the cover in relation to a guide sleeve assembly such that the cover may be rotated to uncover a window. Thus, for covers 510 and 514, the flanges may continue to hold the vessels away from the guide sleeve assembly even when moved to allow access through one of the windows.

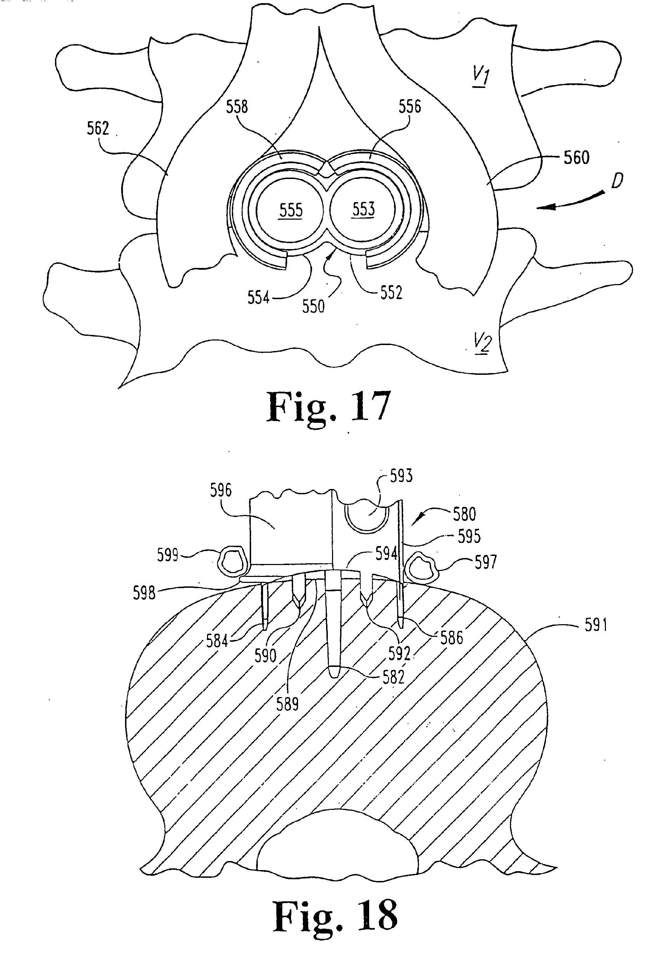

An alternative embodiment shown in Figs. 16a and 16b does not include the enlarged flange 512. Cover 520 has a uniform end 524 and defines an internal channel 522 adapted to receive a guide sleeve assembly. However, in certain surgical procedures it is desirable to use the embodiment having the flange to protect closely adjacent vessels and to urge them away from the distal end of the guide sleeve assembly where it might be possible to contact instruments disposed therein. Without the use of a cover, the guide sleeve assembly may not match the shape of the surface of the vertebral body thereby allowing the potential for contact between instruments in the guide sleeve assembly and closely adjacent vessels. This is particularly dangerous when operating close to the vena cava and aorta. However, as shown in Figs. 17 and 18, the flanges on the covers act as a retractor to urge the vessels away from the guide sleeve assembly.

Referring more specifically to Fig 17, guide sleeve assembly 550 is illustrated as being inserted into a disc space D between two adjacent vertebra V 1 and V2. Disposed adjacent the guide sleeve assembly 550 are vessels 562 and 560 graphically representing portions of the aorta or vena cava. Covers 556 and 558 are mounted on guide tubes 552 and 554, respectively. Flanges on the covers, shown more clearly in Fig. 15a, urge the vessels away from the guide tube and more importantly, away from working channels 553 and 555 were tools would be inserted. Vessels 560 and 562 are most closely adjacent guide tubes 552 and 554 near V₁. Thus, lateral extensions on the guide sleeve assembly may be insufficient to prevent contact between vessels and tools in all applications.

Referring now to Fig. 18, there is shown a top view of a guide sleeve assembly 580 positioned in the disc space adjacent a vertebral body 591. The guide sleeve assembly 580 includes a central distractor 582 and lateral extensions 584 and 586. Spikes 590 and 592 may be inserted into the bone of the vertebral body. For the purposes of illustration, cover 596 has been positioned over a first guide tube, while guide tube 595 with window 593 remains uncovered. Bone engaging end 594 does not entirely conform to vertebra surface 589, thus allowing the possibility of vessel migration into the working channels. Cover 596 with flange 598 urges vessel 599 away from the engagement between bone engaging end 594 and bone surface 589. In contrast, vessel 597 is positioned adjacent the interface between the guide tube and bone, resulting in the potential for vessel migration into the working channel via the space between the bone engaging end 594 and bone surface 589. Thus, covers for use with guide sleeve assemblies according to the present invention may also be useful to further retract vessels away from the interface between the bone engaging end of the guide sleeve assembly and the bone surface.

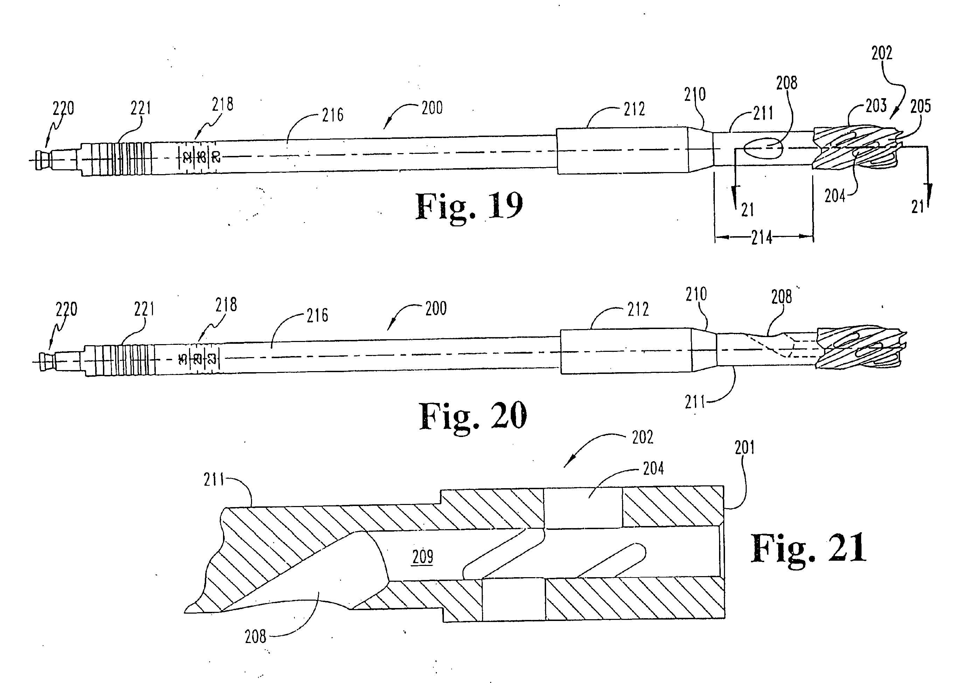

Referring now to Figs. 19 through 21, there is shown a reamer 200. Fig. 20 shows the reamer 200 of Fig. 19 rotated 90 degrees. Reamer 200 includes a cutting head 202 having cutting flutes 203 with troughs 205 disposed therebetween. Disposed in trough 205 is an aperture 204 extending to interior channel 209. A series of apertures 204 are defined in the cutting troughs and communicate with interior channel 209. The interior of cutting head 202 is hollow and forms interior channel 209. Interior channel 209 has a first portion with side walls substantially parallel to the longitudinal axis and a second portion defined by side walls extending at an angle to the longitudinal axis. Preferably the second portion extends at a non-orthogonal angle to permit easy cleaning. The second portion is connected to aperture 208 formed on the outer surface of the shaft and spaced from the cutting head. It will be understood that aperture 208 permits material cut by reaming head 202 to move through the interior channel 209 to exit at aperture 208. Moreover, the reduced diameter segment 211 defines an area between the shaft and guide sleeve assembly where debris from the cutting operation may collect prior to removal of the device. This collection area has a length 214 in a preferred embodiment, although it is understood that this could be extended to increase the volume of material that may be collected. This configuration permits completion of the cutting operation without a requirement to remove the reamer to clean the collected debris. Additionally, the debris may be visualized through guide sleeve assembly windows for evaluation.

Reduced diameter shaft 211 extends proximally to tapered region 210 which expands to a larger diameter guiding portion 212. Tapered region 210 assists ease of insertion and guiding of the shaft of the reamer within a guide sleeve assembly as previously disclosed. Larger diameter guiding portion 212 is sized to have a reasonably close fit within a guide sleeve assembly to permit rotation of the device, yet limit the amount of transverse movement within the guide tube to insure accurate reaming within the bone. Reamer 200 may thereby be guided by a guide sleeve assembly. Shaft 216 interconnects the proximal end to the enlarged area 212.

Disposed on shaft 216 are a series of numbers 218, which indicate the depth the reamer extends into the bone beyond the edge of a cooperable guide sleeve assembly. As can be appreciated from examining Figs. 19 and 20, the numbers are displayed in a stepped arrangement around the circumference of shaft 216. This stepped arrangement permits each number to be larger, preferably three times larger, than they could be if all numbers were listed in a single column along the device. Thus, this arrangement permits easy visualization of the number by the surgeon despite the small incremental adjustment of the device, preferably 1mm increments. Extending more proximally along the shaft 216 are a series of grooves 221 which are adapted to engage a depth stop mechanism (described further below) to adjust the reaming depth of the device. On the proximal end 220 is a Hudson-type connection for engagement with a T-handle or other type of handle.

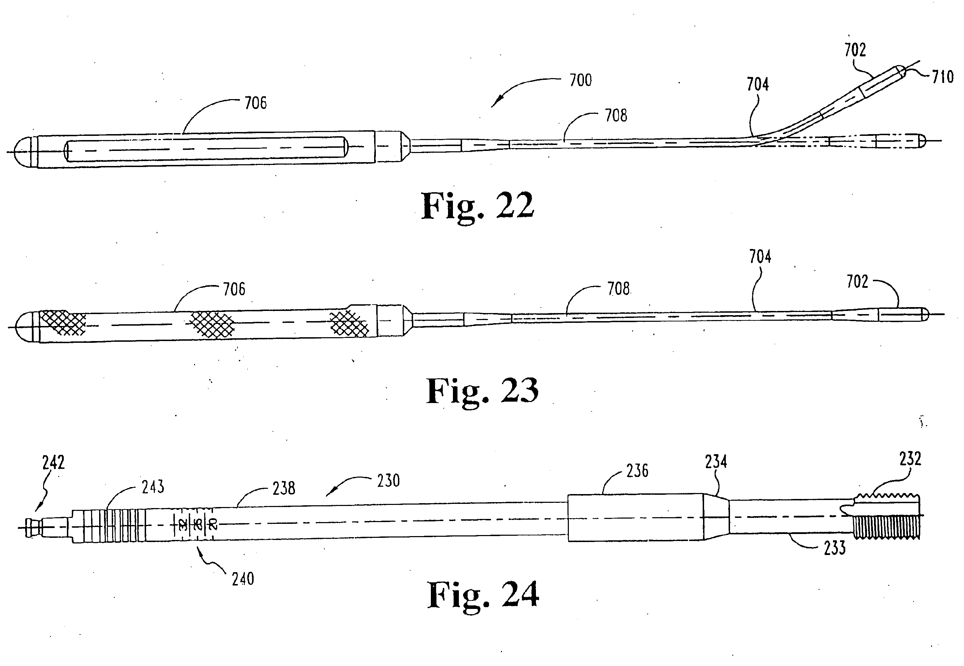

Referring now to Figs. 22 and 23, there is shown a clean out tool 700 adapted for use with the hollow reamer head described above. Clean out tool 700 includes a head 702 having a diameter substantially matching the diameter of internal chamber 209. Clean out tool 700 includes a flexible portion 704. Flexible portion 704 is connected to shaft 708 which is connected to handle 706. Flexible portion 704 allows the device to enter through opening 208 in the reamer and force material out open end 201 of the reamer head as end 710 is advanced. This is an improvement over hollow head reamers that do not provide a clean out channel.

Referring now to Fig. 24, there is shown a thread tap 230 for tapping a reamed out bone space. Tap 230 includes a cutting head 232, and a reduced diameter shaft 233 adjacent head 232 for providing space around the shaft between the guide tube for the collection of debris from the tapping operation. A tapered surface 234 extends to an increased outer diameter area 236. As previously explained with respect to reamer 200, tapered surface 232 permits guiding of the tap within a guide tube and enlarged area 236 by providing a reasonably close fit with the guide tube to maintain the axial alignment of tap 230. Tap 230 includes incrementally stepped depth markings 240 and a Hudson connection 242 as previously disclosed with respect to reamer 200.

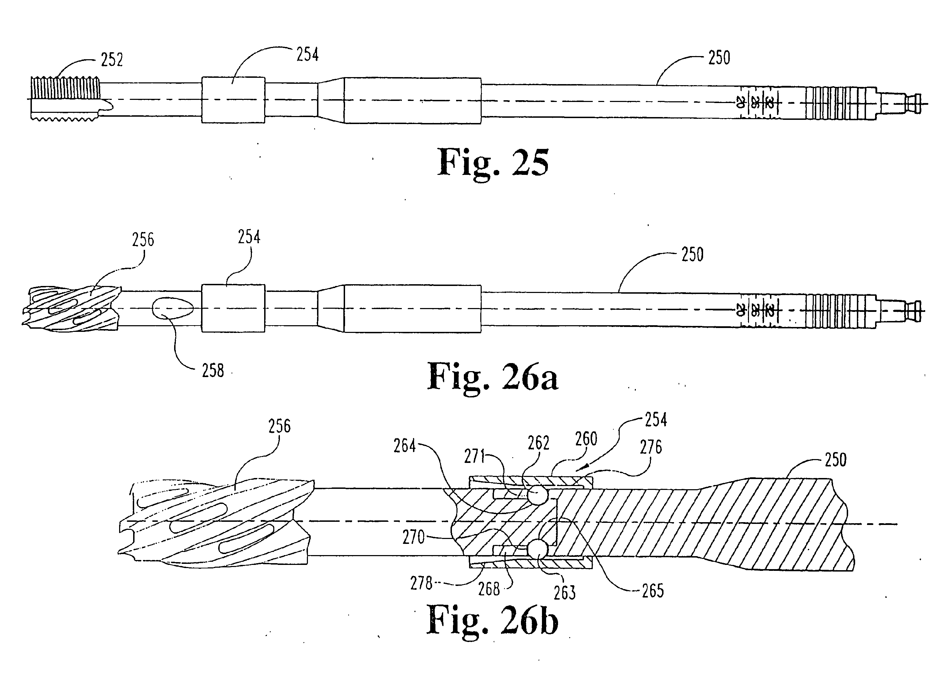

Referring now to Figs. 25 through 26b, there are shown modular cutting tools joined to a shaft. Fig. 25 shows a shaft 250 releasably coupled to tap head 252 by coupler 254. Similarly, shaft 250 is coupled to reamer head 256 by coupler 254. In Fig. 26a reaming head 256 may be removed from shaft 250 at the connection 254. The reamer includes a reaming head 256 having only six cutting apertures disposed around the head and a hollow internal chamber connected to aperture 258. While any number of known connection mechanisms may be used, Fig. 26b shows the use of an axially displaceable collar 260 to release balls 262 and 263 from grooves 264 and 265 of the reamer head. Shaft 250 includes a hollow extension 268 having apertures 270 and 271 to hold balls 263 and 262, respectively. Collar 260 includes a reduced diameter portion 276 adapted to urge balls 262 and 263 into grooves 264 and 265 to lock the cutting head and shaft together. Collar 260 may be axially displaced away from the cutting head to dispose an enlarged internal diameter portion 278 adjacent the balls to allow them to disengage grooves 264 and 265, thereby allowing the cutting head to be disengaged from the shaft. The same mechanism may be used with a variety of cutting heads.

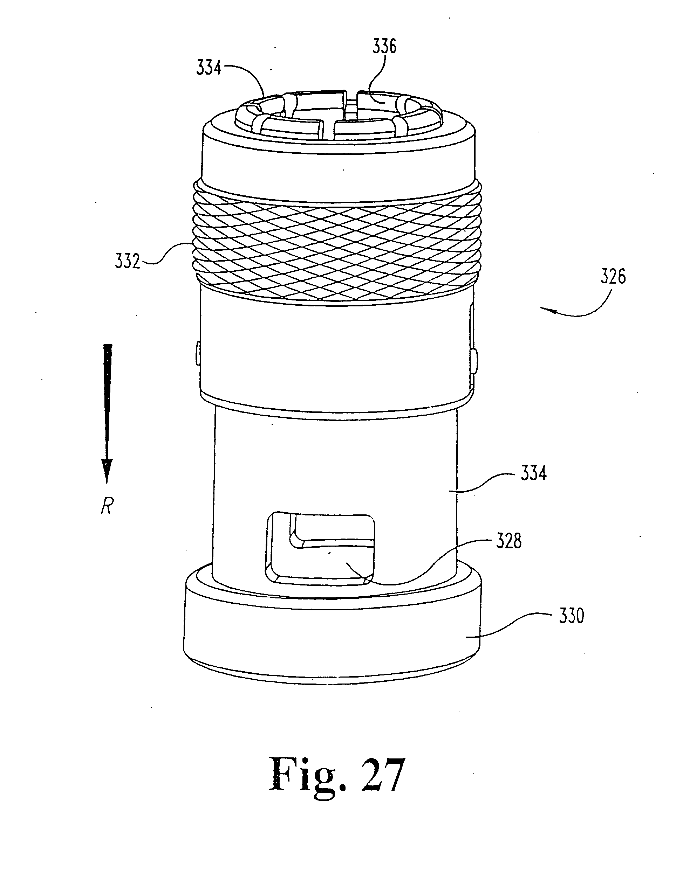

Referring now to Figs. 27 through 31, there is disclosed a depth stop mechanism cooperable with the shaft of a tool and guide tube such as previously disclosed. Such tools can include. without limitation, a reamer, a tap, and an implant inserter. Depth stop 326 includes an enlarged circumferential abutment shoulder 330 adapted to engage the proximal end of a guide tube to prevent further advancement of the stop and any interconnected shaft. Stop 326 further includes viewing windows 328 to permit visualization of depth markings on a shaft extending within the stop. Stop 326 includes a manually operated collar 332 which may be axially displaced to allow flexing of fingers 334. Collar 332 is normally urged into an extended position by spring 342.

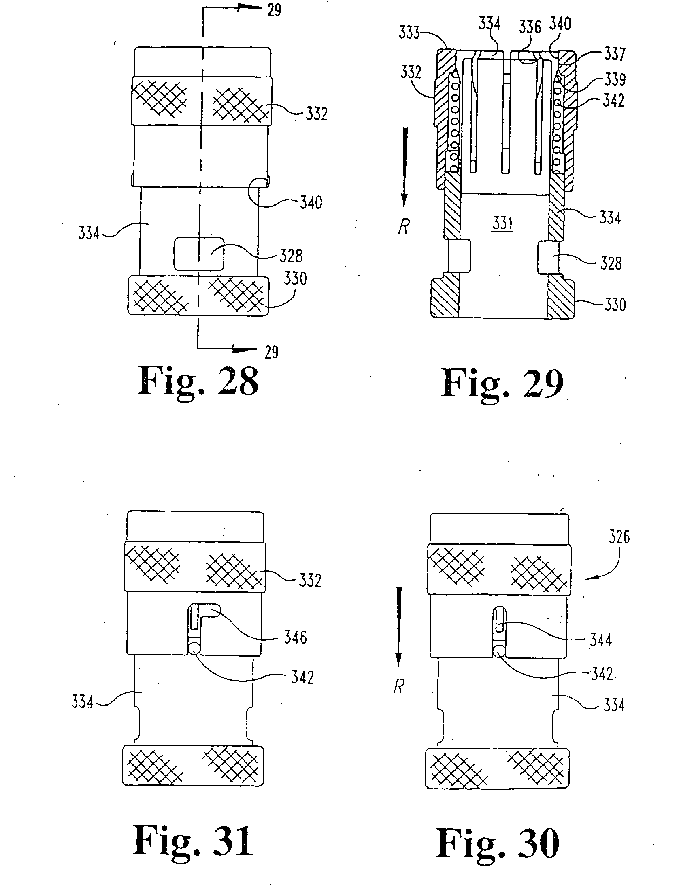

Referring specifically to Fig. 29, fingers 334 include projections 336 extending internally and bearing surface 337 extending externally. The internal projections 336 are configured for engagement within grooves 221 (Fig. 20) defined along a tool shaft of a working tool, and bearing surface 337 is configured to engage collar 332. Additionally, each finger includes an external taper portion 339 adapted for engagement with bearing surface 340 of collar 332 to urge the fingers inwardly as the collar is advanced. It will be understood that in a retracted position, bearing surface 340 of collar 332 will be substantially disengaged from taper 339 and permit fingers 334 to disengage from groove 221 of a working shaft (Fig. 20). With collar 332 in the extended position shown in Fig. 29, bearing surfaces 340 will bear against bearing surface 337 of each finger to urge projections 336 into grooves 221 of a tool shaft. To release fingers, collar 332 may be moved in the direction of arrow R until bearing surface 340 moves beyond tapered surface 339. The flexible fingers may then spring outward. In this manner, a user may quickly and easily disengage the locking mechanism of the stop to advance or retract a working tool and then re-engage the stop at the desired position. Preferably, distal end 333 of collar 332 will extend beyond fingers 334 to limit the possibility that surgical staff may snag protective apparel on exposed fingers.

In a first version shown in Fig. 30, collar 332 is retained on housing 334 by retaining pin 342 extending into the housing and through a slot 344. Retaining pin 342 prevents rotation of collar 332 with respect to housing 334. In an alternate version shown in Fig. 31, collar 332 defines an L-shaped slot 346 which permits axial displacement of collar 332 with respect to body 334, as well as a slight amount of rotation within the slot. It will be understood that the L-shaped slot 346 permits the depth stop mechanism to be locked in a disengaged position which permits free movement of the tool shaft through the depth stop. This is a desirable construction in some instances for easy removal of the depth stop from the tool shaft, as well as for utilization of the tool without the constraints of a depth stop mechanism.

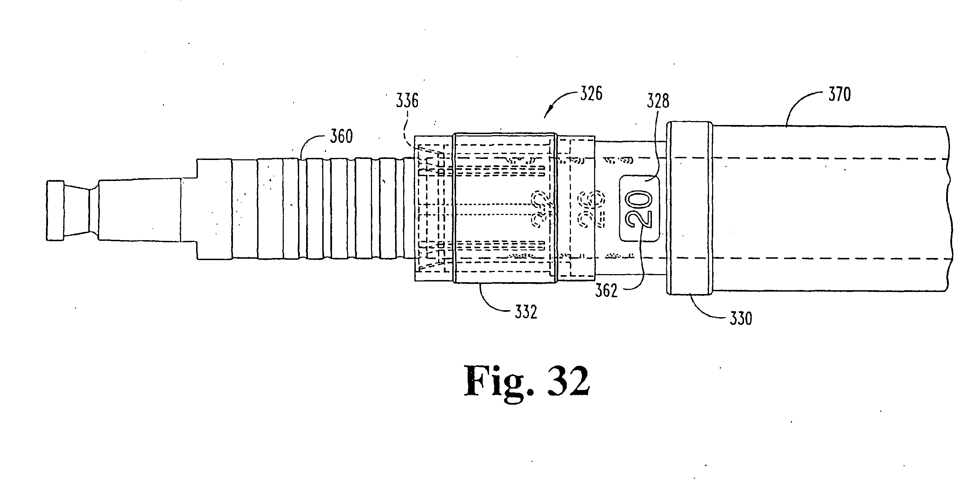

Fig. 32 shows a depth stop 326 engaged with a tool shaft having grooves 360 and marking 362 to show the depth of the distal end of the tool out of the guide tube 370. Abutment shoulder 330 is sized to engage the guide tube to prevent further movement It will be understood that the depth of penetration may be adjusted between a number of positions defined by engagement of the fingers 336 in grooves 360 of the tool shaft. The adjustment is easily accomplished by axial movement of collar 332. Engagement with the tool shaft is indexed by the spacing of grooves 360 on the tool shaft so the exact location of the stop may be easily known. The tool shaft may be rotated with respect to the stop mechanism to display the appropriate depth numeral 362 in window 328.

While the invention has been illustrated and described in detail in the drawings and foregoing description, the same is to be considered as illustrative and not restrictive in character, it being understood that only the preferred embodiments have been shown and described and that all changes and modifications that come within the scope of the invention as defined in the appended claims are desired to be protected.

## Claims

1. A guide sleeve assembly (100), comprising:
a first guide tube (140) defining a first inner working channel (130) opening at a distal bone engaging end of said first guide tube (140) and at least one lateral extension (118) extending from said bone engaging end of said first guide tube (140) having a first height;
a second guide tube (142) defining a second inner working channel (132) opening at a distal bone engaging end of said second guide tube (142) and at least one lateral extension (120) extending from said bone engaging end of said second guide tube (132) having a second height, said first and second lateral extension (118,120) inhibiting encroachment of tissue into an adjacent one of said inner working channels (130, 132);
said first guide tube (140) joined to said second guide tube (142) to define said guide sleeve assembly (100) having a longitudinal axis, said guide sleeve assembly (100) having a proximal portion (102) and an opposite distale portion (104) adapted for insertion into a patient, said proximal portion (102) having a first width and said distal portion having a second width transverse to said longitudinal axis; and
a distractor (116) between said first and second lateral extensions (118, 120), said distractor (116) defining a third height corresponding to a distracted disc space height when inserted into a spiral disc space, **characterized by** said second width being less than said first width and/or said third height being greater than said first and second height.

2. The guide sleeve assembly of claim 1, wherein said first and second bone engaging ends are disposed adjacent one another.

3. The guide sleeve assembly of claim 1, wherein said distractor (116) comprises a central distractor (116) disposed between said first working channel (130) and said second working channel (132) adjacent said first and second bone engaging ends, wherein said central distractor (116) maintains distraction in a disc space when inserted therein.

4. The guide sleeve assembly of claim 3, wherein said central distractor (116) has a thickness greater than a thickness of said first and second lateral extensions (118, 120).

5. The guide sleeve assembly of claim 4, wherein said thickness of said central distractor (116) is approximately twice a wall thickness of said first and second guide tubes (140, 142).

6. The guide sleeve assembly of claim 1, wherein each of said bone engaging ends includes spikes (122, 124, 126) for engaging vertebral bodies.

7. The guide sleeve assembly of claim 1, wherein said first guide tube (140) is oriented substantially parallel to said second guide tube (142).

8. The guide sleeve assembly of claim 1, wherein said first (140) and said second guide tubes (142) each have a wall thickness, said wall thickness of said first and second guide tubes (140, 142) being reduced adjacent said distal portion (104).

9. The guide sleeve assembly of claim 1, wherein said first and second guide tubes (140, 142) each include a circular cross-section.

10. The guide sleeve assembly of claim 1, wherein at least one of said first and second guide tubes (140, 142) includes an outer surface defining at least one window (110, 112; 152, 154, 156, 158) extending from said outer surface to said inner working channel (130, 132), said window (110, 112; 152, 154, 156, 158) permitting visualization of the disc space from outside the at least one guide tube (140, 142) through the inner worming channel (130, 132); and
a cover (160; 182; 510; 514; 520) slidably disposed on said outer surface adjacent said at least one window (110, 112; 152, 154, 156, 158), said cover (160; 182; 510; 514; 520) adapted to slide along an axis of said guide tube (140, 142) to selectively cover said at least one window (110, 112; 152, 154, 156, 158).

11. The guide sleeve assembly of claim 10, wherein said cover (160; 182; 510; 514; 520) includes a dovetail portion (172) slidably disposed within a dovetail groove (168, 169) defined by said outer surface.

12. The guide sleeve assembly of claim 10, wherein said cover (160; 182; 510; 514; 520) is slidable between a first window covering position and a second uncovered position.

13. The guide sleeve assembly of claim 10, wherein said cover (160; 182; 510; 514; 520) is a partial cylinder.

14. The guide sleeve assembly of claim 13, wherein said partial cylinder extends approximately 200°.

15. The guide sleeve assembly of claim 13, wherein said partial cylinder extends approximately 270°.

16. The guide sleeve assembly of claim 10, wherein said cover (160; 182; 510; 514; 520) includes an outwardly extending flange (512; 516), said flange (512; 516) adapted to retract tissue disposed adjacent said outer surface.

17. The guide sleeve assembly of claim 10, wherein said guide tube (140, 142) has a top side and an opposite bottom side, said guide tube (140, 142) having one of said at least one window (110, 112; 152, 154, 156, 158) on each of said top and said bottom side.

18. The guide sleeve assembly of claim 17, wherein said cover (160; 182; 510; 514; 520) selectively covers said one of said at least one window (110, 112; 152, 154, 156, 158) on said bottom side.

19. The guide sleeve assembly of claim 17, wherein said cover (160; 182; 510; 514; 520) selectively covers said one of said at least on window (110, 112; 152, 154, 156, 158) on said top side.

20. The guide sleeve assembly of claim 17, wherein said cover (160; 182; 510; 514; 520) selectively covers said one of said at least one window (110, 112; 152, 154, 156, 158) on each of said top and bottom sides.

21. The guide sleeve assembly of claim 1, wherein each of said first and second guide tubes (140, 142) include an outer surface defining at least one window (110, 112; 152, 154, 156, 158) from said outer surface to said inner working channel (130, 132), said windows (110, 112; 152, 154, 156, 158) permitting visualization of the disc space from outside said first and second guide tubes (140, 142) through the inner working channel thereof (130,132); and
a cover (160; 182; 510; 514; 520) slidably disposed on said outer surfaces of said first and second guide tubes (140, 142) adjacent said at least one window (110, 112; 152, 154, 156, 158) of each of said first and second guide tubes (140, 142), said cover (160; 182; 510; 514; 520) being adapted to slide along said first and second guide tubes (140, 142) to selectively cover said at least one window (110,112; 152, 154, 156,158) thereof.

22. The guide sleeve assembly of claim 21, wherein each of said first and second guide tubes (140, 142) includes two windows (110, 112, 152, 154, 156, 158) spaced along the length thereof.

23. The guide sleeve assembly of claim 22, wherein for each of said first and second guide tubes 140, 142) said two windows (110, 112; 152, 154, 156, 158) are formed in an upper surface of said respective one of said first and second guide tubes (140, 142) said first and second guide tubes (140, 142) further including a second pair of windows (110, 112; 152, 154. 156, 158) included in a lower surface thereof opposite said upper surfaces.

## Patentansprüche

1. Führungshülsenbaugruppe (100), die Folgendes umfasst:
eine erste Führungsröhre (140), die einen ersten inneren Arbeitskanal (130), der sich an einem distalen Knocheneingriffsende der ersten Führungsrohre (140) öffnet, und wenigstens eine seitliche Erweiterung (118), die sich von dem Knocheneingriffsende der ersten Führungsröhre (140) aus erstreckt, definiert, mit einer ersten Höhe,
eine zweite Führungsröhre (142), die einen ersten inneren Arbeitskanal (132), der sich an einem distalen Knocheneingriffsende der zweiten Führungsröhre (142) öffnet, und wenigstens eine seitliche Erweiterung (120), die sich von dem Knocheneingriffsende der zweiten Führungsröhre (142) aus erstreckt, definiert, mit einer zweiten Höhe, wobei die ersten und die zweiten seitlichen Erweiterungen (118, 120) ein Vordringen von Gewebe in einen benachbarte der inneren Arbeitskanäle (130, 132) hemmen,
wobei die erste Führungsröhre (140) mit der zweiten Führungsröhre (142) verbunden ist, um die Führungshülsenbaugruppe (100) mit einer Längsachse zu definieren, wobei die Führungshülsenbaugruppe (100) einen proximalen Abschnitt (102) und einen entgegengesetzten distalen Abschnitt (104), eingerichtet zum Einsetzen in einen Patienten, halt, wobei der proximale Abschnitt (102) eine erste Breite hat und der distale Abschnitt (104) eine zweite Breite hat, quer zu der Längsachse, und
einen Distraktor (116) zwischen den ersten und den zweiten seitlichen Erweiterungen (118, 120), wobei der Distraktor (116) eine dritte Höhe definiert, die einer distrahierten Bandscheibenraumhöhe entspricht, wenn er in einen Bandscheibenraum eingesetzt ist, **dadurch gekennzeichnet, dass** die zweite Breite geringer ist als die erste Breite und/oder die dritte Höhe größer ist als die erste und die zweite Höhe,

2. Führungshülsenbaugruppe nach Anspruch 1, wobei das erste und das zweite Knocheneingriffsende aneinander angrenzend angeordnet sind.

3. Führungshülsenbaugruppe nach Anspruch 1, wobei der Distraktor (116) einen mittigen Distraktor (116) umfasst, der zwischen dem ersten Arbeitskanal (130) und dem zweiten Arbeitskanal (132) an das erste und das zweite Knocheneingriffsende angrenzend angeordnet ist, wobei der mittige Distraktor (116) die Distraktion in einem Bandscheibenraum aufrechterhält, wenn er in denselben eingesetzt ist.

4. Führungshülsenbaugruppe nach Anspruch 3, wobei der mittige Distraktor (116) eine Dicke hat, die größer ist als eine Dicke der ersten und der zweiten seitlichen Erweiterungen (118, 120).

5. Führungshülsenbaugruppe nach Anspruch 4, wobei die Dicke des mittigen Distraktors (116) annähernd doppelt so groß ist wie die Wanddicke der ersten und der zweiten Führungsröhre (140, 142).

6. Führungshülsenbaugruppe nach Anspruch 1, wobei jedes der Knocheneingriffsenden Zacken (122, 124, 126) zum Ineingriffnehmen von Wirbelkörpern einschließt.

7. Führungshülsenbaugruppe nach Anspruch 1, wobei die erste Führungsröhre (140) im Wesentlichen parallel zu der zweiten Führungsröhre (142) ausgerichtet ist.

8. Führungshülsenbaugruppe nach Anspruch 1, wobei die erste (140) und die zweite Führungsröhre (142) jeweils eine Wanddicke haben, wobei die Wanddicke der ersten und der zweiten Führungsröhre (140, 142) an den distalen Abschnitt (104) angrenzend verringert ist.

9. Führungshülsenbaugruppe nach Anspruch 1, wobei die erste und die zweite Führungsröhre (140, 142) jeweils einen kreisförmigen Querschnitt einschließen.

10. Führungshülsenbaugruppe nach Anspruch 1, wobei wenigstens eine der ersten und der zweiten Führungsröhre (140, 142) eine Außenfläche, die wenigstens ein Fenster (110, 112; 152, 154, 156, 158) definiert, das sich von der Außenfläche bis zu dem inneren Arbeitskanal (130, 132) erstreckt, wobei das Fenster (110, 112; 152, 154, 156, 158) ein Sichtbarmachen des Bandscheibenraums von außerhalb der wenigstens einen Führungsröhre (140, 142) durch den inneren Arbeitskanal (130, 132) ermöglicht, und
eine Abdeckung (160; 182; 510; 514; 520), die verschiebbar an der Außenfläche angrenzend an das wenigstens eine Fenster (110, 112; 152, 154, 156, 158) angeordnet ist, einschließt, wobei die Abdeckung (160; 182; 510; 514; 520) dafür eingerichtet ist, längs einer Achse der Führungsröhre (140, 142) zu gleiten, um selektiv das wenigstens eine Fenster (110, 112; 152, 154, 156, 158) abzudecken.

11. Führungshülsenbaugruppe nach Anspruch 10, wobei die Abdeckung (160; 182; 510; 514; 520) einen Schwalbenschwanzabschnitt (172) einschließt, der verschiebbar innerhalb einer Schwalbenschwanzrille (168, 169) angeordnet ist, die durch die Außenfläche definiert wird.

12. Führungshülsenbaugruppe nach Anspruch 10, wobei die Abdeckung (160; 182; 510; 514; 520) zwischen einer ersten, Fenster abdeckenden, Stellung und einer zweiten, nicht abgedeckten, Stellung verschoben werden kann.

13. Führungshülsenbaugruppe nach Anspruch 10, wobei die Abdeckung (160; 182; 510; 514; 520) ein Teilzylinder ist.

14. Führungshülsenbaugruppe nach Anspruch 13, wobei sich der Teilzylinder über annähernd 200° erstreckt.

15. Führungshülsenbaugruppe nach Anspruch 13, wobei sich der Teilzylinder über annähernd 270° erstreckt.

16. Führungshülsenbaugruppe nach Anspruch 10, wobei die Abdeckung (160; 182; 510; 514; 520) einen sich nach außen erstreckenden Flansch (512; 516) einschließt, wobei der Flansch (512; 516) dafür eingerichtet ist, Gewebe zurückzuziehen, das an die Außenfläche angrenzend angeordnet ist.

17. Führungshülsenbaugruppe nach Anspruch 10, wobei die Führungsröhre (140, 142) eine Oberseite und eine entgegengesetzte Unterseite hat, wobei die Führungsröhre (140, 142) sowohl auf der Ober- als auch auf der Unterseite eines von dem wenigstens einen Fenster (110, 112; 152, 154, 156, 158) hat.

18. Führungshülsenbaugruppe nach Anspruch 17, wobei die Abdeckung (160; 182; 510; 514; 520) selektiv das eine von dem wenigstens einen Fenster (110, 112; 152, 154, 156, 158) auf der Unterseite abdeckt.

19. Führungshülsenbaugruppe nach Anspruch 17, wobei die Abdeckung (160; 182; 510; 514; 520) selektiv das eine von dem wenigstens einen Fenster (110, 112; 152, 154, 156, 158) auf der Oberseite abdeckt.

20. Führungshülsenbaugruppe nach Anspruch 17, wobei die Abdeckung (160; 182; 510; 514; 520) selektiv das eine von dem wenigstens einen Fenster (110, 112; 152, 154, 156, 158) sowohl auf der Ober- als auch auf der Unterseite abdeckt.

21. Führungshülsenbaugruppe nach Anspruch 1, wobei sowohl die erste als auch die zweite Führungsröhre (140, 142) eine Außenfläche, die wenigstens ein Fenster (110, 112; 152, 154, 156, 158) von der Außenfläche bis zu dem inneren Arbeitskanal (130, 132) definiert, wobei das Fenster (110, 112; 152, 154, 156, 158) ein Sichtbarmachen des Bandscheibenraums von außerhalb der ersten und der zweiten Führungsröhre (140, 142) durch den inneren Arbeitskanal (130, 132) derselben ermöglicht, und
eine Abdeckung (160; 182; 510; 514; 520), die verschiebbar an den Außenflächen der ersten und der zweiten Führungsröhre (140, 142) angrenzend an das wenigstens eine Fenster (110, 112; 152, 154, 156, 158) sowohl der ersten als auch der zweiten Führungsröhre (140, 142) angeordnet ist einschließt, wobei die Abdeckung (160, 182; 510; 514; 520) dafür eingerichtet ist, längs der ersten und der zweiten Führungsröhre (140, 142) zu gleiten, um selektiv das wenigstens eine Fenster (110, 112; 152, 154, 156, 158) derselben abzudecken.

22. Führungshülsenbaugruppe nach Anspruch 21, wobei sowohl die erste als auch die zweite Führungsröhre (140, 142) zwei Fenster (110, 112; 152, 154, 156, 158) einschließt, die mit Zwischenraum längs der Länge derselben angeordnet sind.

23. Führungshülsenbaugruppe nach Anspruch 22, wobei sowohl für die erste als auch für die zweite Führungsröhre (140, 142) die zwei Fenster (110, 112; 152, 154, 156, 158) in einer oberen Fläche der jeweiligen der ersten und der zweiten Führungsröhre (140, 142) geformt sind, wobei die erste und die zweite Führungsröhre (140, 142) ferner ein zweites Paar von Fenstern (110, 112; 152, 154, 156, 158) einschließen, die in einer unteren Fläche derselben, entgegengesetzt zu der oberen Fläche, eingeschlossen sind.

## Revendications

1. Assemblage de manchon de guidage (100), comprenant :
un premier tube de guidage (140), définissant un premier canal de travail interne (130) ouvert au niveau d'une extrémité distale d'engagement de l'os dudit premier tube de guidage (140), et au moins une extension latérale (118), s'étendant à partir de ladite extrémité d'engagement de l'os dudit premier tube de guidage (140), ayant une première hauteur ;
un deuxième tube de guidage (142), définissant un deuxième canal de travail interne (132), ouvert au niveau d'une extrémité distale d'engagement de l'os dudit deuxième tube de guidage (142), et au moins une extension latérale (120), s'étendant à partir de ladite extrémité d'engagement de l'os dudit deuxième tube de guidage (142), ayant une deuxième hauteur, lesdites première et deuxième extensions latérales (118, 120) empêchant un empiétement de tissus dans un canal adjacent desdits canaux de travail internes (130, 132);
ledit premier tube de guidage (140) étant relié audit deuxième tube de guidage (142) pour définir ledit assemblage de manchon de guidage (100), comportant un axe longitudinal, ledit assemblage de manchon de guidage (100) comportant une partie proximale (102) et une partie distale opposée (104) adaptée pour être insérée dans l'organisme d'un patient, ladite partie proximale (102) ayant une première largeur et la partie distale (104) ayant une deuxième largeur transversale audit axe longitudinal , et
un distracteur (116) entre lesdites première et deuxième extensions latérales (118, 120), ledit distracteur (116) définissant une troisième hauteur correspondant à une hauteur d'un espace discal distracté lors de l'insertion dans un espace discal vertébral, **caractérisé en ce que** ladite deuxième largeur est inférieure à ladite première largeur, et/ou **en ce que** ladite troisième hauteur est supérieure aux dites première et deuxième hauteurs.

2. Assemblage de manchon de guidage selon la revendication 1, dans lequel lesdites première et deuxième extrémités d'engagement de l'os sont agencées en des points mutuellement adjacents.

3. Assemblage de manchon de guidage selon la revendication 1, dans lequel ledit distracteur (116) comprend un distracteur central (116) agencé entre ledit premier canal de travail (130) et ledit deuxième canal de travail (132), en un point adjacent aux dites première et deuxième extrémités d'engagement de l'os, ledit distracteur central (116) maintenant la distraction dans un espace discal lors de son insertion dans celui-ci.

4. Assemblage de manchon de guidage selon la revendication 3, dans lequel ledit distracteur central (116) a une épaisseur supérieure à une épaisseur desdites première et deuxième extensions latérales (118, 120).

5. Assemblage de manchon de guidage selon la revendication 4, dans lequel ladite épaisseur dudit distracteur central (116) représente environ le double d'une épaisseur de paroi desdits premier et deuxième tubes de guidage (140, 142).

6. Assemblage de manchon de guidage selon la revendication 1, dans lequel chacune desdites extrémités d'engagement de l'os englobe des broches (122, 124, 126) destinées à s'engager dans des corps vertébraux.

7. Assemblage de manchon de guidage selon la revendication 1, dans lequel ledit premier tube de guidage (140) est orienté de manière pratiquement parallèle audit deuxième tube de guidage (142).

8. Assemblage de manchon de guidage selon la revendication 1, dans lequel lesdits premier (140) et deuxième (142) tubes de guidage ont chacun une épaisseur de paroi, ladite épaisseur de paroi desdits premier et deuxième tubes de guidage (140, 142) étant réduite près de ladite partie distale (104).

9. Assemblage de manchon de guidage selon la revendication 1, dans lequel lesdits premier et deuxième tubes de guidage (140, 142) englobent chacun une section transversale circulaire.

10. Assemblage de manchon de guidage selon la revendication 1, dans lequel au moins un desdits premier et deuxième tubes de guidage (140, 142) englobe une surface externe définissant au moins une fenêtre (110, 112 ; 152, 154, 156, 158) s'étendant de ladite surface externe vers ledit canal de travail interne (130, 132), ladite fenêtre (110, 112 ; 152, 154, 156, 158) permettant la visualisation de l'espace discal, de l'extérieur du au moins un tube de guidage (140, 142) à travers le canal de travail interne (130, 132) ; et
un couvercle (160 ; 182 ; 510 ; 514 ; 520) agencé de manière coulissante sur ladite surface externe, près de ladite au moins une fenêtre (110, 112 ; 152, 154, 156, 158), ledit couvercle (160; 1.82 ; 510 ; 514: 520) étant adapté pour glisser le long d'un axe dudit tube de guidage (140, 142) poux recouvrir sélectivement ladite au moins une fenêtre (110, 112 ; 152, 154, 156, 158).

11. Assemblage de manchon de guidage selon la revendication 10, dans lequel ledit couvercle (160; 182 ; 510 ; 514 ; 520) englobe une partie en queue d'aronde (172), agencée de manière coulissante dans une rainure en queue d'aronde (168, 169) définie par ladite surface externe.

12. Assemblage de manchon de guidage selon la revendication 10, dans lequel ledit couvercle (160 ; 182 ; 510 ; 514 ; 520) peut glisser entre une première position recouvrant la fenêtre et une deuxième position sans recouvrement.

13. Assemblage de manchon de guidage selon la revendication 10, dans lequel ledit couvercle (160 ; 182 ; 510 ; 514 ; 520) est constitué par un cylindre partiel.

14. Assemblage de manchon de guidage selon la revendication 13, dans lequel ledit cylindre partiel s'étend sur environ 200°.

15. Assemblage de manchon de guidage selon la revendication 13, dans lequel ledit cylindre partiel s'étend sur environ 270°.

16. Assemblage de manchon de guidage selon la revendication 10, dans lequel ledit couvercle (160 ; 182 ; 510 ; 514 ; 520) englobe une bride s'étendant vers l'extérieur (512 ; 516), ladite bride (512 ; 516) étant adaptée pour rétracter les tissus situés près de ladite surface externe.

17. Assemblage de manchon de guidage selon la revendication 10, dans lequel ledit tube de guidage (140, 142) comporte un côté supérieur et un côté inférieur opposé, ledit tube de guidage (140, 142) comportant une fenêtre de ladite au moins une fenêtre (110, 112; 152, 154, 156, 158) sur chacun desdits côtés supérieur et inférieur.

18. Assemblage de manchon de guidage selon la revendication 17, dans lequel ledit couvercle (160 ; 182; 510 ; 514 ; 520) recouvre sélectivement ladite une fenêtre de ladite au moins une fenêtre (110,112; 152, 154, 156, 158) sur ledit côté inférieur.

19. Assemblage de manchon de guidage selon la revendication 17, dans lequel ledit couvercle (160 ; 182 ; 510 ; 514 ; 520) recouvre sélectivement ladite une fenêtre de ladite au moins une fenêtre (110, 112 ; 152, 154, 156, 158) sur ledit coté supérieur.

20. Assemblage de manchon de guidage selon la revendication 17, dans lequel ledit couvercle (160 ; 182 ; 510 ; 514 ; 520) recouvre sélectivement ladite une fenêtre de ladite au moins une fenêtre (110, 112 ; 152, 154, 156, 158) sur chacun desdits côtés supérieur et inférieur.

21. Assemblage de manchon de guidage selon la revendication 1. dans lequel chacun desdits premier et deuxième tubes de guidage (140, 142) englobe une surface externe définissant au moins une fenêtre (110, 112;152, 154, 156, 158), de ladite surfaces externe vers ledit canal de travail interne (130, 132), lesdites fenêtres (110, 112 ; 152, 154, 156, 158) permettant la visualisation de l'espace discal, de l'extérieur desdits premier et deuxième tubes de guidage (140, 142) à travers le canal de travail interne (130, 132) de ceux-ci; et
un couvercle (160 ; 182 ; 510 ; 514 ; 520), agencé de manière coulissante sur lesdites surfaces externes desdits premier et deuxième tubes de guidage (140, 142), près de ladite au moins une fenêtre (110, 112 ; 152, 154, 156, 158) de chacun desdits premier et deuxième tubes de guidage (140, 142), ledit couvercle (160; 182 ; 510 ; 514 ; 520) étant adapté pour glisser le long desdits premier et deuxième tubes de guidage (140, 142), pour recouvrir sélectivement ladite au moins une fenêtre (110, 112 ; 152, 154, 156, 158) de ceux-ci.

22. Assemblage de manchon de guidage selon la revendication 21, dans lequel chacun desdits premier et deuxième tubes de guidage (140, 142) englobe deux fenêtres (110, 112 ; 152, 154, 156, 158) espacées le long de leur longueur.

23. Assemblage de manchon de guidage selon la revendication 22, dans lequel lesdites deux fenêtres (110, 112 ; 152, 154, 156, 158) sont formées pour chacun desdits premier et deuxième tubes de guidage (140, 142) dans une surface supérieure d'un dit tube respectif desdits premier et deuxième tubes de guidage (140, 142), lesdits premier et deuxième tubes de guidage (140, 142) englobant en outre une deuxième paire de fenêtres (110, 112 ; 152, 154, 156, 158) incluse dans une surface inférieure de ceux-ci, opposées à ladite surface supérieure.
